(19) Europäisches Patentamt
European Patent Office
Office européen des brevets

(11) Veröffentlichungsnummer: **0 467 183 A1**

(12) **EUROPÄISCHE PATENTANMELDUNG**

(21) Anmeldenummer: **91111297.7**

(22) Anmeldetag: **06.07.91**

(51) Int. Cl.[5]: **C07C 311/53**, A01N 47/24, A01N 41/06

(30) Priorität: **20.07.90 DE 4023040**

(43) Veröffentlichungstag der Anmeldung: **22.01.92 Patentblatt 92/04**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Ooms, Pieter, Dr.**
**Doerperhofstrasse 16**
**W-4150 Krefeld 1(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R. Dr.**
**Im Waldwinkel 110**
**W-5060 Bergisch Gladbach 2(DE)**

(54) **Herbizide auf Basis von sulfonylierten (Thio)Carbamidsäureestern, neue sulfonylierte Carbamidsäureester und Verfahren zu deren Herstellung.**

(57) Die Erfindung betrifft die herbizide Verwendung von neuen und bekannten sulfonylierten (Thio)-Carbamidsäureestern der Formel (I)

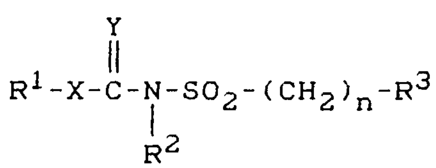

(I)

sowie neue sulfonylierte Carbamidsäureester der Formel (Ia)

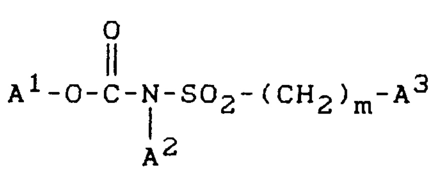

(Ia)

und Verfahren zu deren Herstellung.

EP 0 467 183 A1

Die vorliegende Erfindung betrifft die Verwendung von teilweise bekannten sulfonylierten (Thio)-Carbamidsäureestern als Herbizide, neue sulfonylierte Carbamidsäureester und Verfahren zu deren Herstellung.

Es ist bekannt, daß bestimmte sulfonylierte Carbamidsäureester herbizide Eigenschaften aufweisen (vergleiche Nature 207 (1965), 655-656; US-P 3 377 375; US-P 3 512 955; J. Sci. Food Agr. 1968 (Suppl.), 3-7 - zitiert in Chem. Abstracts 70, 56548x; US-P 3 799 760; US-P 3 849 110; US-P 3 933 894; US-P 4 213 775; JP-A 76-15621 - zitiert in Chem. Abstracts 84, 175167w; JP-A 82-126404 - zitiert in Chem. Abstracts 97, 177032r). Die Herbizidwirkung dieser Verbindungen ist jedoch nicht ganz zufriedenstellend.

Weiter sind bestimmte sulfonylierte (Thio)Carbamidsäureester als Antidote für Herbizide, als Zwischenprodukte für Herbizide oder als Fungizide bekannt (vergleiche US-P 4 021 229; US-P 4 168 152; DE-OS 2 644 446; DE-OS 2 644 504; EP-A 101 407; EP-A 127 469; EP-A 269 141; EP-A 298 679; JP-A 82-140763 - zitiert in Chem. Abstracts 98, 53400k; JP-A 82-203059 - zitiert in Chem. Abstracts 99, 38221e; JP-A 85-214785 - zitiert in Chem. Abstracts 104, 109685u).

Die vorliegende Erfindung betrifft die herbizide Verwendung der teilweise bekannten sulfonylierten (Thio)Carbamidsäureester der allgemeinen Formel (I)

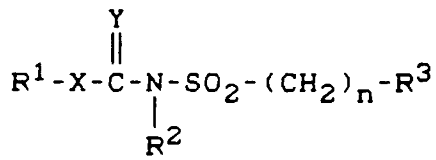

(I)

in welcher

- n für die Zahlen 0, 1 oder 2 steht,
- $R^1$ für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,
- $R^2$ für Wasserstoff oder für jeweils gegebenenfalls substituiertes Alkyl, Alkenyl, Alkinyl, Cycloalkyl, Cycloalkylalkyl, Aryl, Arylalkyl, Heterocyclyl, Heterocyclylalkyl, für ein Metalläquivalent, für Ammonium, Alkylammonium, Dialkylammonium oder Trialkylammonium steht oder zusammen mit $R^1$ für gegebenenfalls substituiertes Alkandiyl steht,
- $R^3$ für mindestens einmal in ortho-Position substituiertes Phenyl oder für jeweils gegebenenfalls substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht,
- X für Sauerstoff oder Schwefel steht und
- Y für Sauerstoff oder Schwefel steht,

wobei die Verbindungen ausgenommen sind, in denen

(a) $R^3$ für 2-Nitro-phenyl steht, welches zusätzlich substituiert sein kann und

(b) $R^3$ für 2-Methyl-phenyl oder 2-Methoxycarbonyl-phenyl steht und $R^1$ für Methyl oder Ethyl steht, n für die Zahl 0, $R^2$ für Wasserstoff und X und Y für Sauerstoff stehen.

Die erfindungsgemäß zu verwendenden Verbindungen der Formel (I) zeigen bei guter Verträglichkeit gegenüber Kulturpflanzen, wie Weizen, Gerste, Mais und Soja, starke Wirkung gegen Unkräuter.

In der Definition von $R^1$, $R^2$ und $R^3$ bzw. in den weiter unten angegebenen Definitionen von $A^1$, $A^2$ und $A^3$ sind aliphatische Reste, wie Alkyl, Alkoxy, Alkenyl oder Alkinyl, allein oder in Zusammensetzungen, wie Halogenalkyl, Halogenalkenyl, Alkoxyalkyl, jeweils geradkettig oder verzweigt, auch wenn dies nicht ausdrücklich erwähnt ist.

Halogen, allein oder in Zusammensetzungen wie Halogenalkyl, bedeutet Fluor, Chlor, Brom, Iod, vorzugsweise Fluor, Chlor oder Brom.

Die Erfindung betrifft Herbizide vorzugsweise auf Basis von Verbindungen der Formel (I), in welcher

- n für die Zahlen 0, 1 oder 2 steht,
- $R^1$ für Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatommen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen

steht, oder $R^1$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen, oder $R^1$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $R^1$ für jeweils gegebenenfalls einfach bis vierfach, gleich oder verschieden substituiertes Heterocyclyl oder Heterocylylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, oder Halogenalkyl, Halogenalkoxy oder Halogenalkylthio mit jeweils 1 bis 4 Kohlenstoffatomen und 1 bis 9 gleichen oder verschiedenen Halogenatomen,

$R^2$ für Wasserstoff, für Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, oder $R^2$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen, Alkoxycarbonyl mit bis zu 4 Kohlenstoffatomen, oder $R^2$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $R^2$ für jeweils gegebenenfalls einfach bis vierfach, gleich oder verschieden substituiertes Heterocyclyl oder Heterocyclylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und 1 bis 9 gleichen oder verschiedenen Halogenatomen, oder $R^2$ für ein Alkalimetall- oder ein Erdalkalimetall-Äquivalent, für Ammonium, $C_1$-$C_4$-Alkylammonium, Di-($C_1$-$C_4$-Alkyl)-ammonium oder Tri-($C_1$-$C_4$-Alkyl)-ammonium steht oder zusammen mit $R^1$ für gegebenenfalls durch Methyl oder Ethyl substituiertes geradkettiges Alkandiyl mit 2 bis 5 Kohlenstoffatomen steht,

$R^3$ für mindestens einmal in ortho-Position und insgesamt einfach bis dreifach substituiertes Phenyl oder für jeweils mindestens einfach und gegebenenfalls bis dreifach substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht, wobei jeweils als Substituenten ausgewählt sind: Halogen, Cyano, Alkyl, Halogenalkyl, Alkoxyalkyl, Alkoxy, Halogenalkoxy, Alkoxyalkoxy, Alkylthio, Halogenalkylthio, Alkylsulfinyl, Halogenalkylsulfinyl, Alkylsulfonyl oder Halogenalkylsulfonyl mit jeweils 1 bis 4 Kohlenstoffatomen in den Alkylteilen und gegebenenfalls 1 bis 9 Halogenatomen, Alkenyl, Halogenalkenyl, Alkinyl oder Halogenalkinyl mit jeweils 2 bis 4 Kohlenstoffatomen und gegebenenfalls 1 bis 7 Halogenatomen, Dialkylaminosulfonyl mit 1 oder 2 Kohlenstoffatomen in den Alkylteilen, Phenyl, Alkoxycarbonyl oder Halogenalkoxycarbonyl mit jeweils 1 bis 5 Kohlenstoffatomen und gegebenenfalls 1 bis 3 Halogenatomen im Alkoxyteil, sowie Dialkylaminocarbonyl mit 1 oder 2 Kohlenstoffatomen in den Alkylteilen,

X für Sauerstoff oder Schwefel steht und

Y für Sauerstoff oder Schwefel steht,

wobei die obengenannten Verbindungen ausgenommen sind.

Insbesondere werden erfindungsgemäß die Verbindungen der Formel (I) verwendet, in welcher

n für die Zahlen 0 oder 1 steht,

$R^1$ für Methyl, Ethyl, n-Propyl, Isopropyl, n-Butyl, Isobutyl, sec-Butyl, tert-Butyl, Allyl, Propargyl, für jeweils geradkettiges oder verzweigtes Pentyl, Hexyl, Butenyl, Pentenyl, Hexenyl, Butinyl, Pentinyl, Hexinyl oder Heptinyl, für Halogenalkyl, Halogenalkenyl oder Halogenalkinyl mit bis zu 5 Kohlenstoffatomen, letztere mit insbesondere 3 oder 4 Kohlenstoffatomen, und jeweils 1 bis 3 Halogenatomen, insbesondere Fluor- und/oder Chloratomen, für Cyanalkyl, Alkoxyalkyl oder Alkoxycarbonylalkyl mit jeweils 1 bis 4 Kohlenstoffatomen in den einzelnen Alkylteilen, für jeweils gegebenenfalls einfach bis dreifach, gleich oder verschieden durch Fluor, Chlor, Brom, Cyano, Methyl, Ethyl, n- oder iso-Propyl, Ethenyl, Propenyl, Ethinyl, Propinyl, Methoxycarbonyl und Ethoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls einfach bis dreifach, gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl, oder für jeweils gegebenenfalls einfach oder zweifach, gleich oder verschieden durch Fluor, Chlor, Brom, Methyl, Ethyl, Trifluormethyl, Methoxy, Ethoxy und Difluormethoxy substituiertes Furyl, Furylmethyl, Thienyl, Thienylmethyl, Oxazolyl, Oxazolylmethyl, Isoxazolyl, Isoxazolylmethyl, Thiazolyl, Thiazolylmethyl, Isothiazolyl, Isothiazolylmethyl, Oxadiazolyl, Thiadiazolyl, Pyridinyl, Pyrimidinyl, Oxadiazolylmethyl, Thiadiazolylmethyl, Pyridinylmethyl, Pyrimidinylmethyl, Tetrahydrofuranylmethyl oder Perhydrofuranylmethyl steht,

$R^2$ für Wasserstoff, Methyl, Ethyl, n-Propyl, Isopropyl, n-Butyl, Isobutyl, Allyl, Propargyl, für jeweils geradkettiges oder verzweigtes Pentyl, Hexyl, Butenyl, Pentenyl, Hexenyl, Butinyl, Pentinyl, Hexinyl oder Heptinyl, für Halogenalkyl, Halogenalkenyl oder Halogenalkinyl mit bis zu 5 Kohlenstoffatomen, letztere mit insbesondere 3 oder 4 Kohlenstoffatomen, und jeweils 1 bis 3 Halogenatomen, insbesondere Fluor- und/oder Chloratomen, für Cyanalkyl, Alkoxyalkyl oder Alkoxycarbonylalkyl mit jeweils 1 bis 4 Kohlenstoffatomen in den einzelnen Alkylteilen, für jeweils gegebenenfalls einfach bis dreifach, gleich oder verschieden durch Fluor, Chlor, Brom, Methyl, Ethyl, n-Propyl, Ethenyl, Propenyl, Ethinyl, Propinyl, Methoxycarbonyl, Ethoxycarbonyl und Cyano substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls einfach bis dreifach, gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl steht, oder für Natrium, Kalium, Ammonium, Methylammonium, Ethylammonium, n-Propylammonium, Isopropylammonium, Dimethylammonium, Diethylammonium, Di-n-propylammonium, Diisopropylammonium, Trimethylammonium, Triethylammonium oder Tri-n-propylammonium steht, oder zusammen mit $R^1$ für Dimethylen ($-CH_2CH_2-$) oder Trimethylen ($-CH_2CH_2CH_2-$) steht,

$R^3$ für in ortho-Position durch Fluor, Chlor, Brom, Cyano, Methyl, Ethyl, Trifluormethyl, Methoxy, Ethoxy, Difluormethoxy, Trifluormethoxy, Fluorethoxy, Chlorethoxy, Methoxyethoxy, Ethoxyethoxy, Methylthio, Ethylthio, Methylsulfinyl, Ethylsulfinyl, Methylsulfonyl, Ethylsulfonyl, Dimethylaminosulfonyl, Phenyl, Methoxycarbonyl, Ethoxycarbonyl, n-Propoxycarbonyl, Isopropoxycarbonyl, n-Butoxycarbonyl, Neopentyloxycarbonyl, Chlorethoxycarbonyl oder Dimethylaminocarbonyl substituiertes Phenyl steht, welches gegebenenfalls einen weiteren Fluor-, Chlor-oder Brom-Substituenten enthält, oder für Pyridinyl steht, welches durch Fluor, Chlor, Brom, Methoxycarbonyl, Ethoxycarbonyl, Dimethylaminocarbonyl, Methylsulfonyl oder Ethylsulfonyl substituiert ist, oder für Thienyl steht, welches durch Chlor, Brom, Methoxycarbonyl oder Ethoxycarbonyl substituiert ist, oder für Pyrazolyl steht, welches durch Chlor, Brom, Methyl, Ethyl, Phenyl, Methoxycarbonyl und/oder Ethoxycarbonyl substituiert ist,

X für Sauerstoff oder Schwefel steht und

Y für Sauerstoff oder Schwefel steht,

wobei die obengenannten Verbindungen ausgenommen sind.

Ganz besonders bevorzugt ist die erfindungsgemäße Verwendung von Verbindungen der Formel (I), in

welcher

- n für die Zahlen 0 oder 1 steht,
- $R^1$ die oben als insbesondere bevorzugt angegebene Bedeutung hat,
- $R^2$ für Wasserstoff, Methyl, Ethyl, n-Propyl, Isopropyl, Allyl, Propargyl oder Methylpropargyl steht,
- $R^3$ die oben als insbesondere bevorzugt angegebene Bedeutung hat,
- X für Sauerstoff oder Schwefel steht und
- Y für Sauerstoff steht,

wobei die obengenannten Verbindungen ausgenommen sind.

Die im folgenden hervorgehobenen Bedeutungen gelten sowohl für die erfindungsgemäße Verwendung der Verbindungen der Formel (I) als auch für die unten beschriebenen neuen Stoffe der Formel (Ia).

Besonders hervorzuheben ist die Zahl 0 für n. Die Bedeutung von Wasserstoff für $R^2$ bzw. $A^2$ ist besonders hervorzuheben.

- X steht insbesondere für Sauerstoff.
- Y steht insbesondere für Sauerstoff.

Besonders hervorzuheben ist die Bedeutung von 2-Methoxycarbonylphenyl, 2-Ethoxycarbonylphenyl und 2-Neopentyloxycarbonylphenyl für $R^3$ bzw. $A^3$.

Beispiele für die erfindungsgemäß zu verwendenden Verbindungen der Formel (I) sind in der nachstehenden Tabelle 1 aufgeführt.

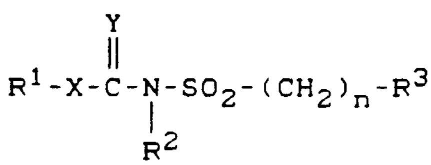

(I)

Tabelle 1: Beispiele für die Verbindungen der Formel (I)

| $R^1$ | $R^2$ | $R^3$ | n | X | Y |
|---|---|---|---|---|---|
| $H_3C$ | H | 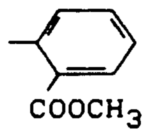 | 0 | O | S |
| $H_3C$ | H | 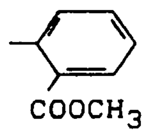 | 0 | S | O |
| $H_3C$ | H | 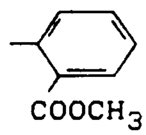 | 0 | S | S |
| $H_3C$ | H | 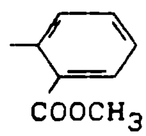 | 1 | S | O |
| $H_3C$ | H | 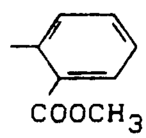 | 1 | O | S |
| $H_5C_2$ | H | 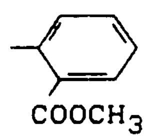 | 1 | S | O |
| $H_7C_3$ | H | 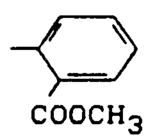 | 1 | O | O |
| $H_9C_4$ | H | 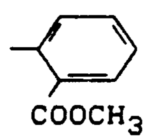 | 1 | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_2C{=}CH-CH_2-$ | H | 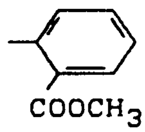 | 1 | O | O |
| $H_3C-CH{=}CH-CH_2-$ | H | 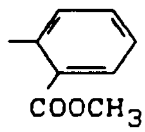 | 1 | O | O |
| $(H_3C)_2C{=}CH-CH_2-$ | H | 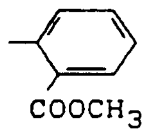 | 1 | O | O |
| $(H_3C)_2C{=}CH-CH_2-$ | H | 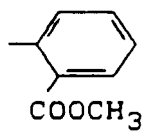 | 0 | O | O |
| $H_3C-CH{=}CH-CH_2-$ | H | 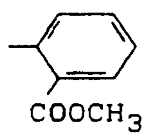 | 0 | S | O |
| $H_3C-C{\equiv}C-CH_2-$ | H | 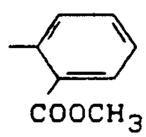 | 0 | S | O |
| $H_3C-C{\equiv}C-CH_2-$ | H | 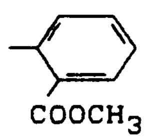 | 2 | O | O |
| $H_3C$ | H | 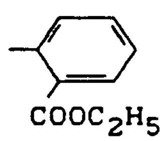 | 0 | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_3C$ | H | 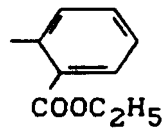 | O | S | O |
| $H_3C$ | H | 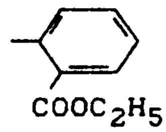 | O | S | S |
| $H_5C_2$ | H | 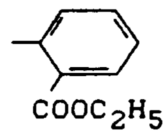 | O | O | O |
| $H_5C_2$ | H | 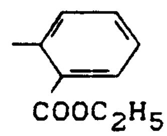 | O | S | O |
| $H_5C_2$ | H | 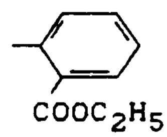 | 1 | O | O |
| $H_5C_2$ | H | 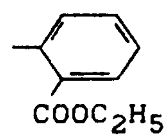 | 1 | S | O |
| $H_5C_2$ | H | 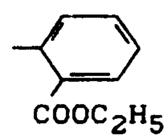 | O | O | S |
| $H_2C{=}CH-CH_2-$ | H | 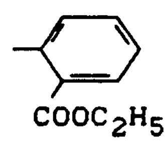 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_3C-CH{=}CH-CH_2-$ | H | 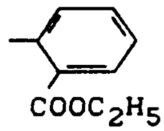 | O | O | O |
| $HC\equiv C-CH_2-$ | H | 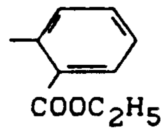 | O | O | O |
| $ClCH_2-C\equiv C-CH_2-$ | H | 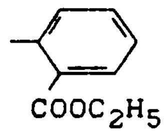 | O | O | O |
| $H_5C_2-C\equiv C-CH_2-$ | H | 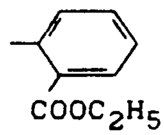 | O | O | O |
| $H_3C-C\equiv C-CH_2CH_2-$ | H | 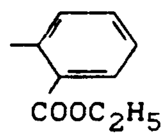 | O | O | O |
| $(H_3C)_2C{=}CH-CH_2-$ | H | 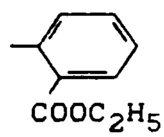 | O | O | O |
| $HC\equiv C-CH-$<br>\|<br>$CH_3$ | H | 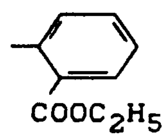 | O | O | O |
| 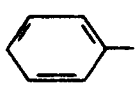 | H | 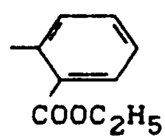 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| 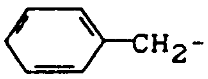 | H | 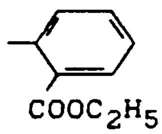 | O | O | O |
| 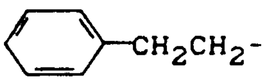 | H | 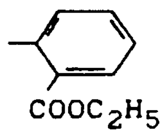 | O | O | O |
| 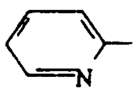 | H | 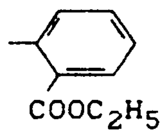 | O | O | O |
| 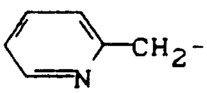 | H | 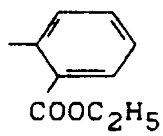 | O | O | O |
| 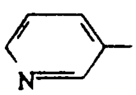 | H | 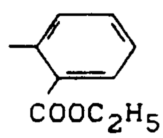 | O | O | O |
| 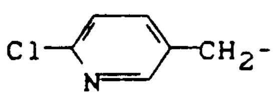 | H | 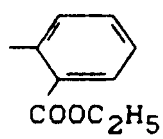 | O | O | O |
| 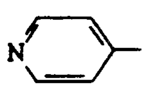 | H | 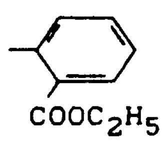 | O | O | O |
| 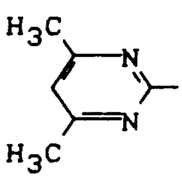 | H | 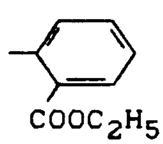 | O | S | O |

<u>Tabelle 1:</u> Beispiele für die Verbindungen der Formel (I) (Fortsetzung)

| $R^1$ | $R^2$ | $R^3$ | n | X | Y |
|---|---|---|---|---|---|
| 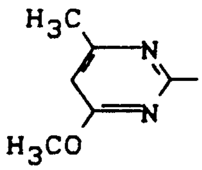 | H | 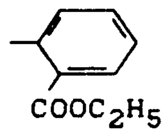 | 1 | O | O |
| 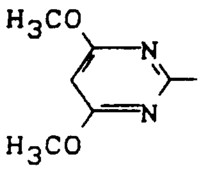 | H | 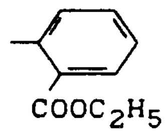 | O | O | O |
| $H_3C$ | H | 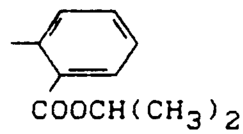 | O | O | O |
| $H_5C_2$ | H | 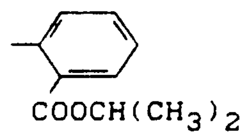 | O | S | O |
| $H_7C_3$ | H | 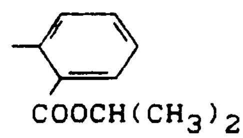 | 1 | O | O |
| $H_3C-CH{=}CH-CH_2-$ | H | 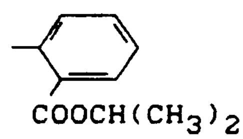 | O | O | O |
| $ClCH_2-C{\equiv}C-CH_2-$ | H | 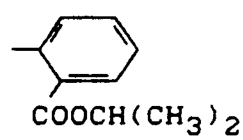 | O | O | O |
| $H_3C$ | H | 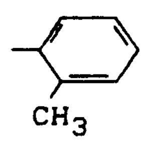 | O | S | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_5C_2$ | H | 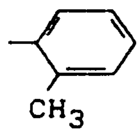 | 0 | S | 0 |
| $H_2C{=}CH-CH_2-$ | H | 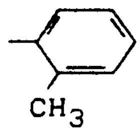 | 0 | 0 | 0 |
| $H_3C-CH{=}CH-CH_2$ | H | 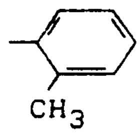 | 0 | S | 0 |
| $HC{\equiv}C-CH_2-$ | H | 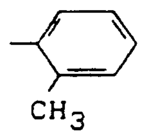 | 0 | 0 | 0 |
| $H_3C-C{\equiv}C-CH_2-$ | H | 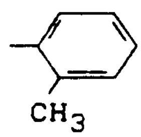 | 0 | S | 0 |
| $ClCH_2-C{\equiv}C-CH_2-$ | H | 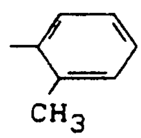 | 0 | 0 | 0 |
| 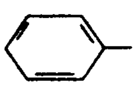 | H | 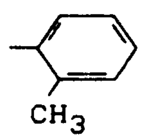 | 0 | 0 | 0 |
| 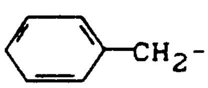 | H | 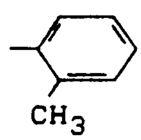 | 0 | S | 0 |

Tabelle 1: Beispiele für die Verbindungen der Formel (I) (Fortsetzung)

| $R^1$ | $R^2$ | $R^3$ | n | X | Y |
|---|---|---|---|---|---|
| 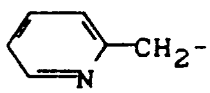 | H | 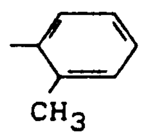 | 0 | O | O |
| 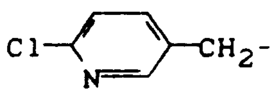 | H | 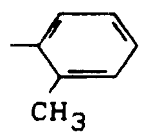 | 0 | O | O |
| 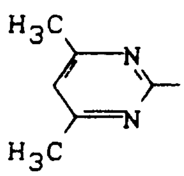 | H | 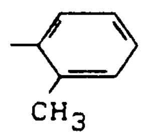 | 0 | O | O |
| $H_3C$ | H | 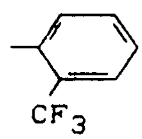 | 0 | S | O |
| $H_5C_2$ | H | 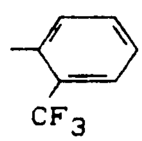 | 0 | S | O |
| $H_2C{=}CH{-}CH_2{-}$ | H | 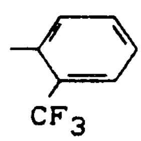 | 0 | O | O |
| $H_3C{-}CH{=}CH{-}CH_2$ | H | 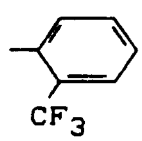 | 0 | S | O |
| $HC{\equiv}C{-}CH_2{-}$ | H | 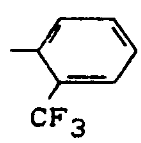 | 0 | O | O |

Tabelle 1: Beispiele für die Verbindungen der Formel (I) (Fortsetzung)

| $R^1$ | $R^2$ | $R^3$ | n | X | Y |
| --- | --- | --- | --- | --- | --- |
| $H_3C-C{\equiv}C-CH_2-$ | H | 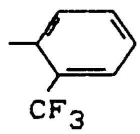 | 0 | S | 0 |
| $ClCH_2-C{\equiv}C-CH_2-$ | H | 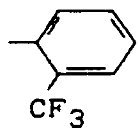 | 0 | 0 | 0 |
| 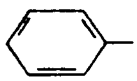 | H | 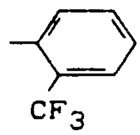 | 0 | 0 | 0 |
| 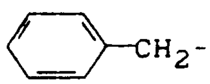 | H | 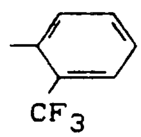 | 0 | S | 0 |
| 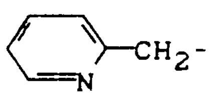 | H | 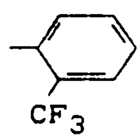 | 0 | 0 | 0 |
| 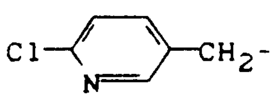 | H | 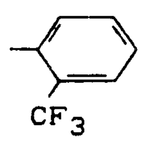 | 0 | 0 | 0 |
| 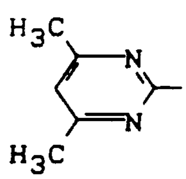 | H | 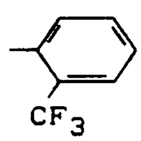 | 0 | 0 | 0 |
| $H_3C$ | H | 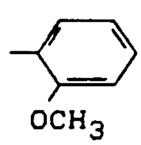 | 0 | S | 0 |

**Tabelle 1:** Beispiele für die Verbindungen der Formel (I) (Fortsetzung)

| $R^1$ | $R^2$ | $R^3$ | n | X | Y |
|---|---|---|---|---|---|
| $H_5C_2$ | H | 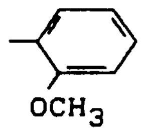 | O | S | O |
| $H_2C{=}CH{-}CH_2{-}$ | H | 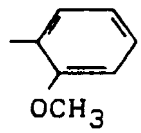 | O | O | O |
| $H_3C{-}CH{=}CH{-}CH_2$ | H | 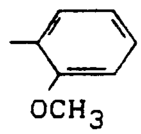 | O | S | O |
| $HC{\equiv}C{-}CH_2{-}$ | H | 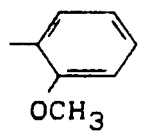 | O | O | O |
| $H_3C{-}C{\equiv}C{-}CH_2{-}$ | H | 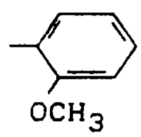 | O | S | O |
| $ClCH_2{-}C{\equiv}C{-}CH_2{-}$ | H | 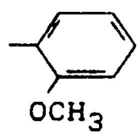 | O | O | O |
| 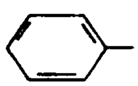 | H | 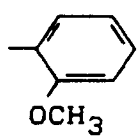 | O | O | O |
| 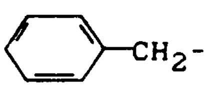 | H | 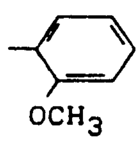 | O | S | O |

<u>Tabelle 1:</u> Beispiele für die Verbindungen der Formel (I) (Fortsetzung)

| $R^1$ | $R^2$ | $R^3$ | n | X | Y |
|---|---|---|---|---|---|
| 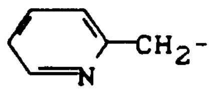 | H | 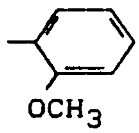 | O | O | O |
| 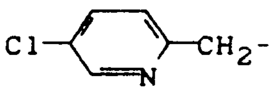 | H | 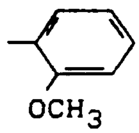 | O | O | O |
| 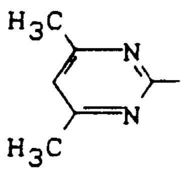 | H | 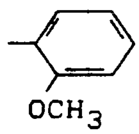 | O | O | O |
| $H_3C$ | H | 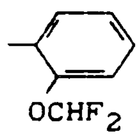 | O | S | O |
| $H_5C_2$ | H | 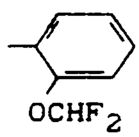 | O | S | O |
| $H_2C{=}CH\text{-}CH_2\text{-}$ | H | 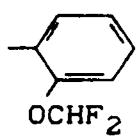 | O | O | O |
| $H_3C\text{-}CH{=}CH\text{-}CH_2\text{-}$ | H | 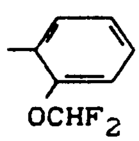 | O | S | O |
| $HC{\equiv}C\text{-}CH_2\text{-}$ | H | 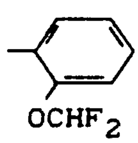 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_3C-C\equiv C-CH_2-$ | H | 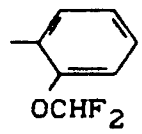 | 0 | S | O |
| $ClCH_2-C\equiv C-CH_2-$ | H | 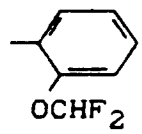 | 0 | O | O |
| 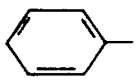 | H | 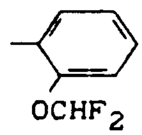 | 0 | O | O |
| 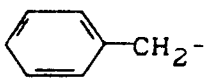 | H | 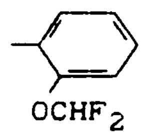 | 0 | S | O |
| 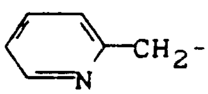 | H | 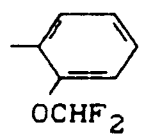 | 0 | O | O |
| 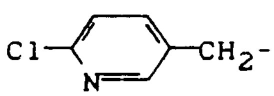 | H | 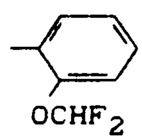 | 0 | O | O |
| 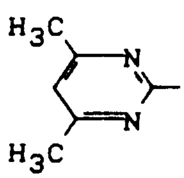 | H | 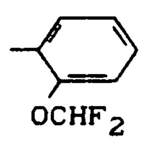 | 0 | O | O |
| $H_3C$ | H | 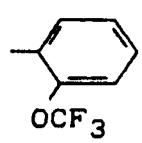 | 0 | S | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_5C_2$ | H | 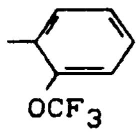 | O | S | O |
| $H_2C{=}CH-CH_2-$ | H | 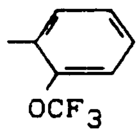 | O | O | O |
| $H_3C-CH{=}CH-CH_2$ | H | 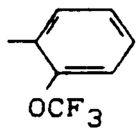 | O | S | O |
| $HC{\equiv}C-CH_2-$ | H | 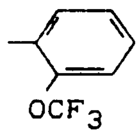 | O | O | O |
| $H_3C-C{\equiv}C-CH_2-$ | H | 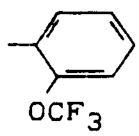 | O | S | O |
| $ClCH_2-C{\equiv}C-CH_2-$ | H | 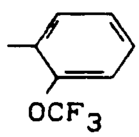 | O | O | O |
| 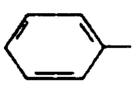 | H | 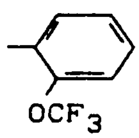 | O | O | O |
| 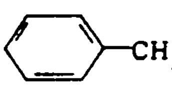 $CH_2-$ | H | 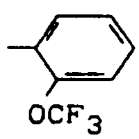 | O | S | O |

Tabelle 1: Beispiele für die Verbindungen der Formel (I) (Fortsetzung)

| $R^1$ | $R^2$ | $R^3$ | n | X | Y |
|---|---|---|---|---|---|
| 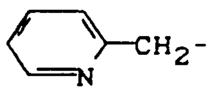 | H | 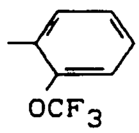 | O | O | O |
| 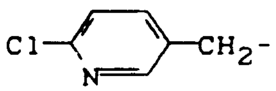 | H | 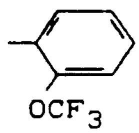 | O | O | O |
| 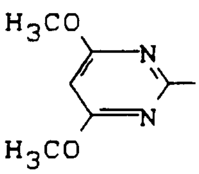 | H | 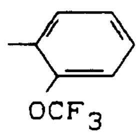 | O | O | O |
| $H_3C$ | H | 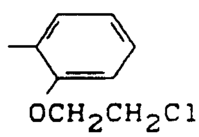 | O | S | O |
| $H_5C_2$ | H | 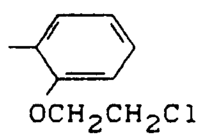 | O | S | O |
| $H_2C=CH-CH_2-$ | H | 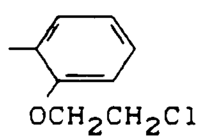 | O | O | O |
| 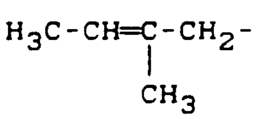 | H | 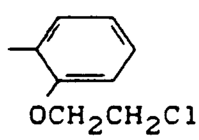 | O | S | O |
| $HC\equiv C-CH_2-$ | H | 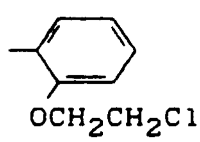 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_3C-C{\equiv}C-CH_2-$ | H | 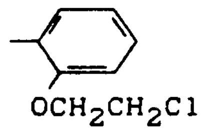 | O | S | O |
| $ClCH_2-C{\equiv}C-CH_2-$ | H | 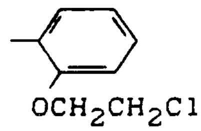 | O | O | O |
| 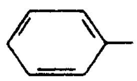 | H | 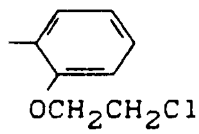 | O | O | O |
| 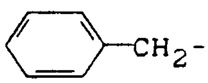 | H | 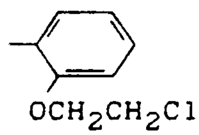 | O | S | O |
| 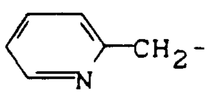 | H | 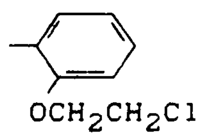 | O | O | O |
| 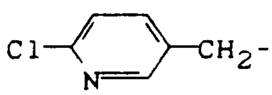 | H | 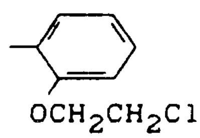 | O | O | O |
| 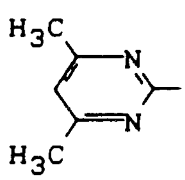 | H | 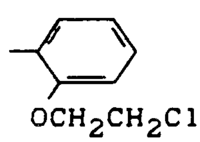 | O | O | O |
| $H_3C$ | H | 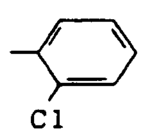 | O | S | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_5C_2$ | H | 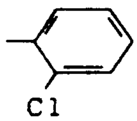 | O | S | O |
| $H_2C{=}CH{-}CH_2{-}$ | H | 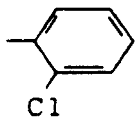 | O | O | O |
| $H_3C{-}CH{=}CH{-}CH_2$ | H | 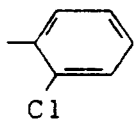 | O | S | O |
| $HC{\equiv}C{-}CH_2{-}$ | H | 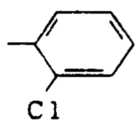 | O | O | O |
| $H_3C{-}C{\equiv}C{-}CH_2{-}$ | H | 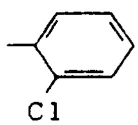 | O | S | O |
| $ClCH_2{-}C{\equiv}C{-}CH_2{-}$ | H | 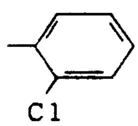 | O | O | O |
| 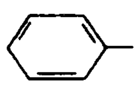 | H | 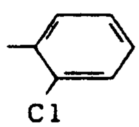 | O | O | O |
| 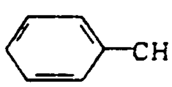 $CH_2{-}$ | H | 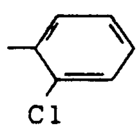 | O | S | O |

Tabelle 1: Beispiele für die Verbindungen der Formel (I) (Fortsetzung)

| $R^1$ | $R^2$ | $R^3$ | n | X | Y |
|---|---|---|---|---|---|
| 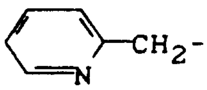 | H | 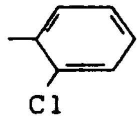 | 0 | O | O |
| 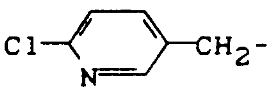 | H | 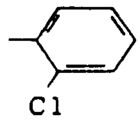 | 0 | O | O |
| 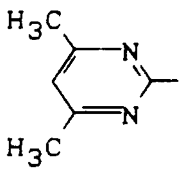 | H | 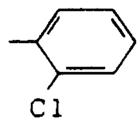 | 0 | O | O |
| $H_3C$ | H | 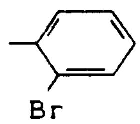 | 0 | S | O |
| $H_5C_2$ | H | 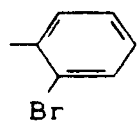 | 0 | S | O |
| $H_2C=CH-CH_2-$ | H | 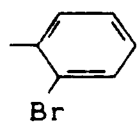 | 0 | O | O |
| $H_3C-CH=CH-CH_2-$ | H | 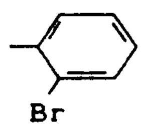 | 0 | S | O |
| $HC\equiv C-CH_2-$ | H | 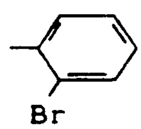 | 0 | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_3C-C{\equiv}C-CH_2-$ | H | 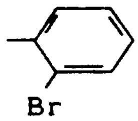 | 0 | S | 0 |
| $ClCH_2-C{\equiv}C-CH_2-$ | H | 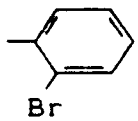 | 0 | 0 | 0 |
| 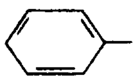 | H | 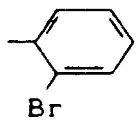 | 0 | 0 | 0 |
| 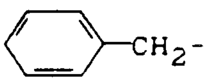 | H | 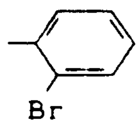 | 0 | S | 0 |
| 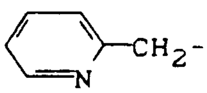 | H | 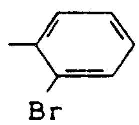 | 0 | 0 | 0 |
| 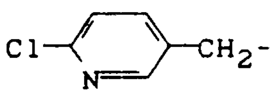 | H | 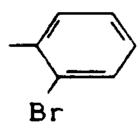 | 0 | 0 | 0 |
| 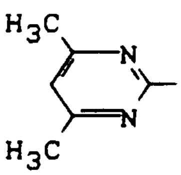 | H | 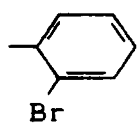 | 0 | 0 | 0 |
| $H_3C$ | H | 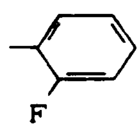 | 0 | S | 0 |

Tabelle 1: Beispiele für die Verbindungen der Formel (I)
(Fortsetzung)

| $R^1$ | $R^2$ | $R^3$ | n | X | Y |
|---|---|---|---|---|---|
| $H_5C_2$ | H | 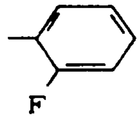 | 0 | S | O |
| $H_2C{=}CH-CH_2-$ | H | 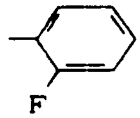 | 0 | O | O |
| $H_3C-CH{=}CH-CH_2$ | H | 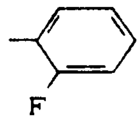 | 0 | S | O |
| $HC{\equiv}C-CH_2-$ | H | 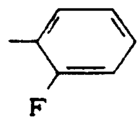 | 0 | O | O |
| $H_3C-C{\equiv}C-CH_2-$ | H | 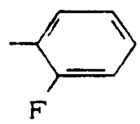 | 0 | S | O |
| $ClCH_2-C{\equiv}C-CH_2-$ | H | 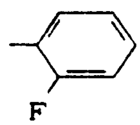 | 0 | O | O |
| 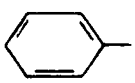 | H | 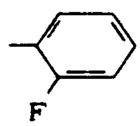 | 0 | O | O |
| 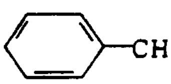 $CH_2-$ | H | 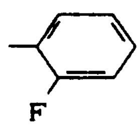 | 0 | S | O |

Tabelle 1: Beispiele für die Verbindungen der Formel (I) (Fortsetzung)

| $R^1$ | $R^2$ | $R^3$ | n | X | Y |
|---|---|---|---|---|---|
| 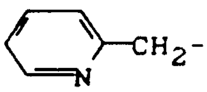 | H | 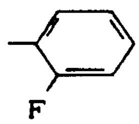 | O | O | O |
| 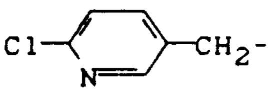 | H | 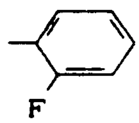 | O | O | O |
| 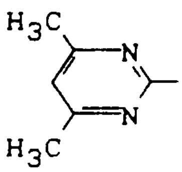 | H | 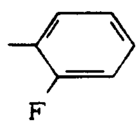 | O | O | O |
| $H_3C$ | H | 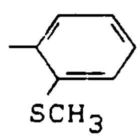 | O | S | O |
| $H_5C_2$ | H | 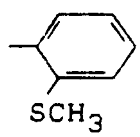 | O | S | O |
| $H_2C=CH-CH_2-$ | H | 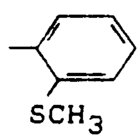 | O | O | O |
| $H_3C-CH=CH-CH_2-$ | H | 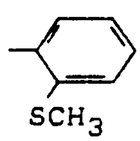 | O | S | O |
| $HC\equiv C-CH_2-$ | H | 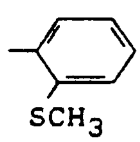 | O | O | O |

<u>Tabelle 1:</u> Beispiele für die Verbindungen der Formel (I)
(Fortsetzung)

| $R^1$ | $R^2$ | $R^3$ | n | X | Y |
|---|---|---|---|---|---|
| $H_3C-C\equiv C-CH_2-$ | H | 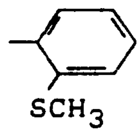 | O | S | O |
| $ClCH_2-C\equiv C-CH_2-$ | H | 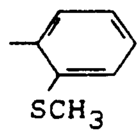 | O | O | O |
| 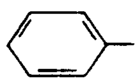 | H | 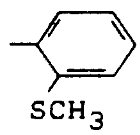 | O | O | O |
| 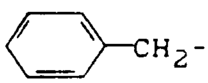 | H | 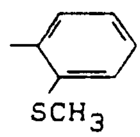 | O | S | O |
| 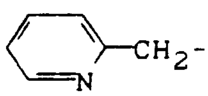 | H | 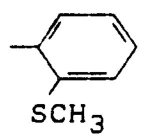 | O | O | O |
| 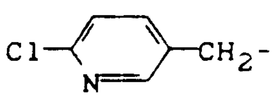 | H | 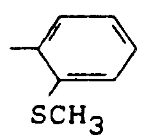 | O | O | O |
| 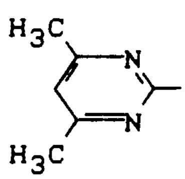 | H | 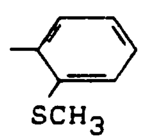 | O | O | O |
| $H_3C$ | H | 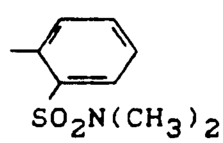 | O | S | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_5C_2$ | H | 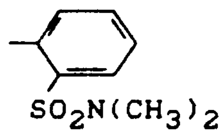 | O | S | O |
| $H_2C{=}CH\text{-}CH_2\text{-}$ | H | 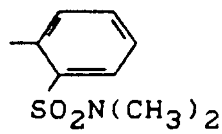 | O | O | O |
| $H_3C\text{-}CH{=}CH\text{-}CH_2$ | H | 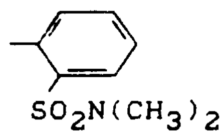 | O | S | O |
| $HC{\equiv}C\text{-}CH_2\text{-}$ | H | 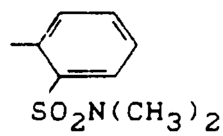 | O | O | O |
| $H_3C\text{-}C{\equiv}C\text{-}CH_2\text{-}$ | H | 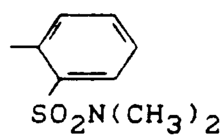 | O | S | O |
| $ClCH_2\text{-}C{\equiv}C\text{-}CH_2\text{-}$ | H | 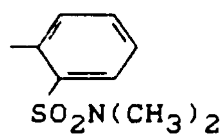 | O | O | O |
| 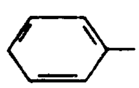 | H | 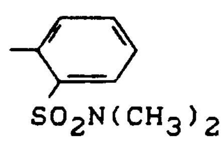 | O | O | O |
| 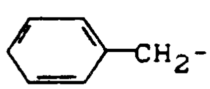 | H | 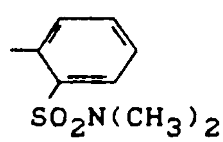 | O | S | O |

Tabelle 1: Beispiele für die Verbindungen der Formel (I) (Fortsetzung)

| $R^1$ | $R^2$ | $R^3$ | n | X | Y |
|---|---|---|---|---|---|
| 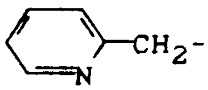 | H | 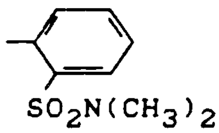 | 0 | O | O |
| 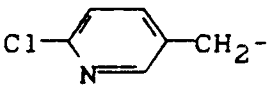 | H | 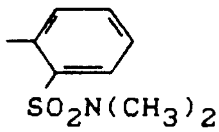 | 0 | O | O |
| 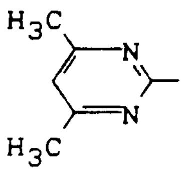 | H | 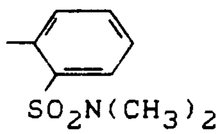 | 0 | O | O |
| $H_3C$ | H | 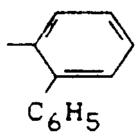 | 0 | S | O |
| $H_5C_2$ | H | 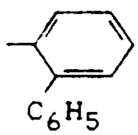 | 0 | S | O |
| $H_2C=CH-CH_2-$ | H | 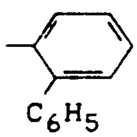 | 0 | O | O |
| $H_3C-CH=CH-CH_2-$ | H | 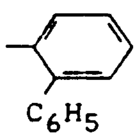 | 0 | S | O |
| $HC\equiv C-CH_2-$ | H | 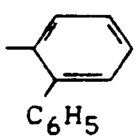 | 0 | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_3C-C\equiv C-CH_2-$ | H | 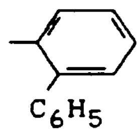 | O | S | O |
| $ClCH_2-C\equiv C-CH_2-$ | H | 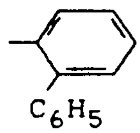 | O | O | O |
| 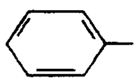 | H | 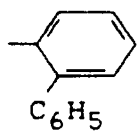 | O | O | O |
| 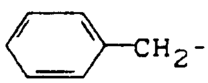 | H | 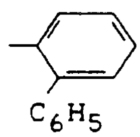 | O | S | O |
| 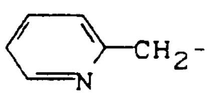 | H | 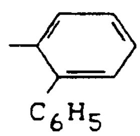 | O | O | O |
| 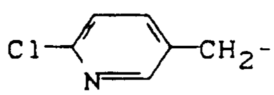 | H | 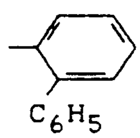 | O | O | O |
| 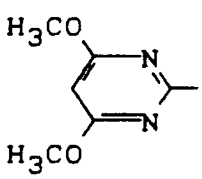 | H | 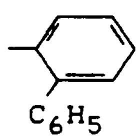 | O | O | O |
| $H_3C$ | H | 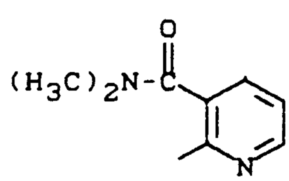 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $H_5C_2$ | H | 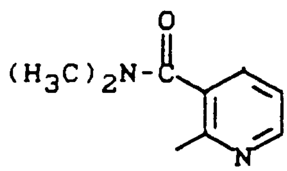 | 0 | S | 0 |
| $H_7C_3$ | H | 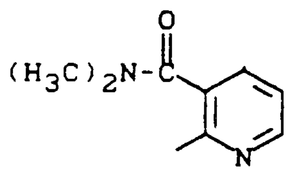 | 0 | 0 | 0 |
| $H_2C{=}CH-CH_2-$ | H | 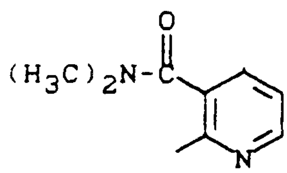 | 0 | 0 | 0 |
| $H_3C-CH{=}CH-CH_2$ | H | 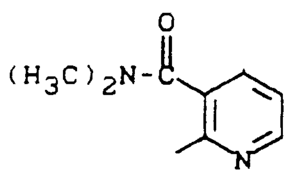 | 0 | 0 | 0 |
| $HC{\equiv}C-CH_2-$ | H | 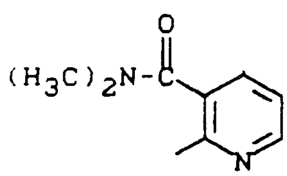 | 0 | 0 | 0 |
| $H_3C-C{\equiv}C-CH_2-$ | H | 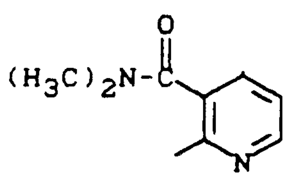 | 0 | 0 | 0 |
| $ClCH_2-C{\equiv}C-CH_2-$ | H | 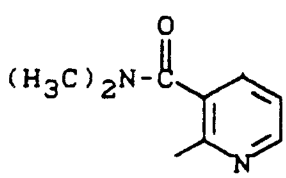 | 0 | 0 | 0 |

<u>Tabelle 1:</u> Beispiele für die Verbindungen der Formel (I) (Fortsetzung)

| $R^1$ | $R^2$ | $R^3$ | n | X | Y |
|---|---|---|---|---|---|
| 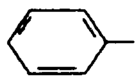 | H | 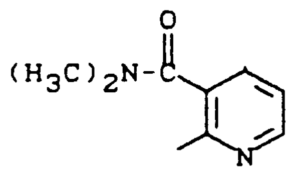 | O | O | O |
| 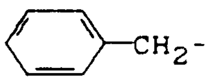 | H | 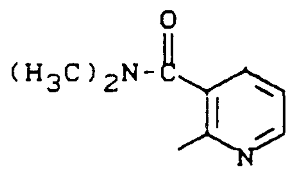 | O | O | O |
| 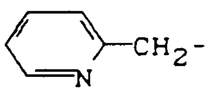 | H | 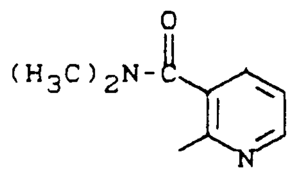 | O | O | O |
| 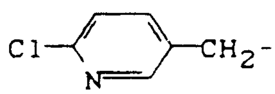 | H | 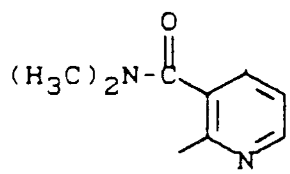 | O | O | O |
| 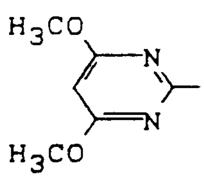 | H | 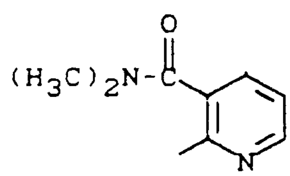 | O | O | O |
| $H_3C$ | H | 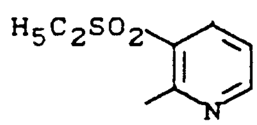 | O | O | O |
| $H_5C_2$ | H | 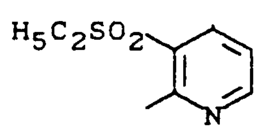 | O | S | O |

Tabelle 1: Beispiele für die Verbindungen der Formel (I)
(Fortsetzung)

| $R^1$ | $R^2$ | $R^3$ | n | X | Y |
|---|---|---|---|---|---|
| $H_7C_3$ | H | 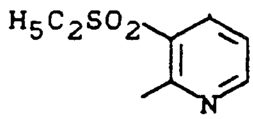 | O | O | O |
| $H_2C=CH-CH_2-$ | H | 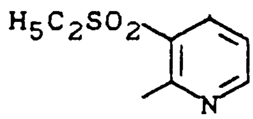 | O | O | O |
| $H_3C-CH=CH-CH_2-$ | H | 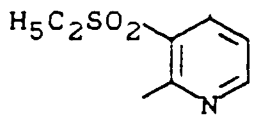 | O | O | O |
| $HC\equiv C-CH_2-$ | H | 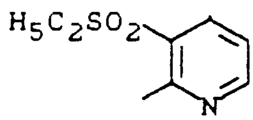 | O | O | O |
| $H_3C-C\equiv C-CH_2-$ | H | 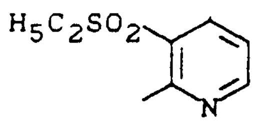 | O | O | O |
| $ClCH_2-C\equiv C-CH_2-$ | H | 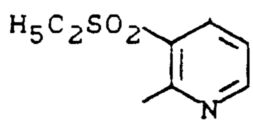 | O | O | O |
| 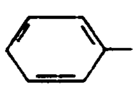 | H | 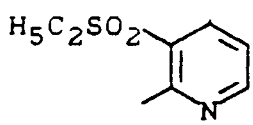 | O | O | O |
| 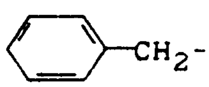 | H | 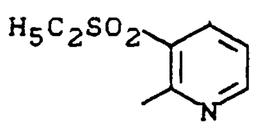 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| 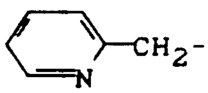 | H | 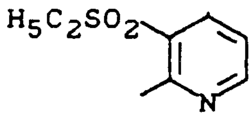 | O | O | O |
| 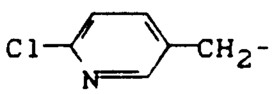 | H | 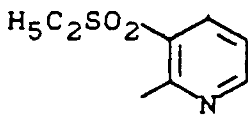 | O | O | O |
| 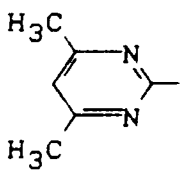 | H | 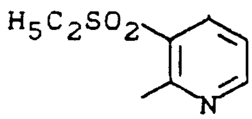 | O | O | O |
| $H_3C$ | H | 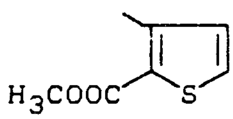 | O | O | O |
| $H_5C_2$ | H | 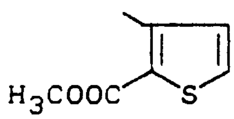 | O | S | O |
| $H_7C_3$ | H | 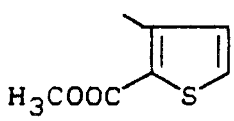 | O | O | O |
| $H_3C-CH=CH-CH_2-$ | H | 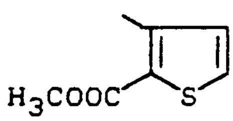 | O | O | O |
| $(H_3C)_2C=CH-CH_2-$ | H | 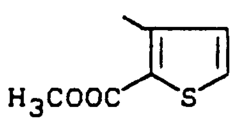 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| $HC\equiv C-CH_2-$ | H | 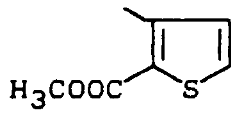 | O | O | O |
| $H_3C-C\equiv C-CH_2-$ | H | 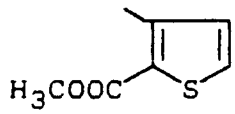 | O | O | O |
| $ClCH_2-C\equiv C-CH_2-$ | H | 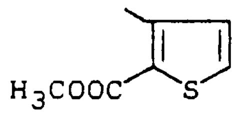 | O | O | O |
| 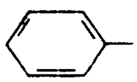 | H | 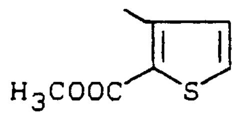 | O | O | O |
| 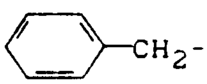 | H | 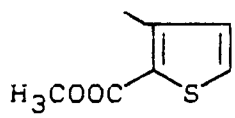 | O | O | O |
| 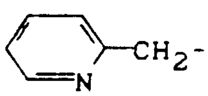 | H | 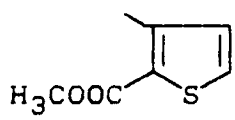 | O | O | O |
| 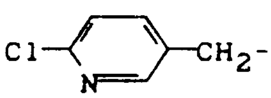 | H | 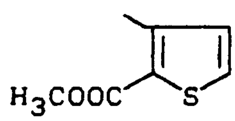 | O | O | O |
| 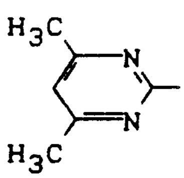 | H | 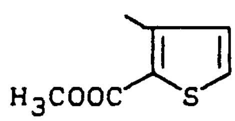 | O | O | O |

<u>Tabelle 1:</u> Beispiele für die Verbindungen der Formel (I)
(Fortsetzung)

| $R^1$ | $R^2$ | $R^3$ | n | X | Y |
|---|---|---|---|---|---|
| $H_3C$ | H | 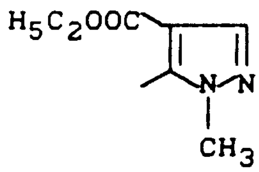 | O | O | O |
| $H_5C_2$ | H | 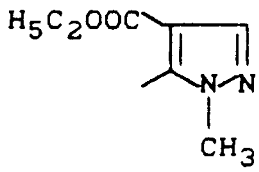 | O | S | O |
| $H_7C_3$ | H | 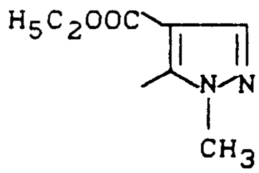 | O | O | O |
| $H_2C{=}CH{-}CH_2{-}$ | H | 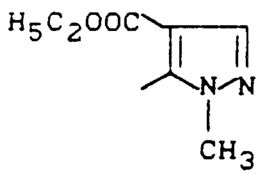 | O | O | O |
| $H_3C{-}CH{=}CH{-}CH_2{-}$ | H | 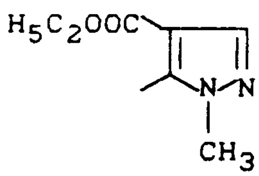 | O | O | O |
| $(H_3C)_2C{=}CH{-}CH_2{-}$ | H | 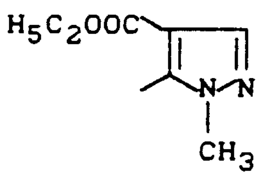 | O | O | O |
| $HC{\equiv}C{-}CH_2{-}$ | H | 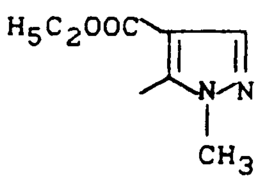 | O | O | O |

Tabelle 1: Beispiele für die Verbindungen der Formel (I) (Fortsetzung)

| $R^1$ | $R^2$ | $R^3$ | n | X | Y |
|---|---|---|---|---|---|
| 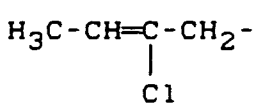 | H | 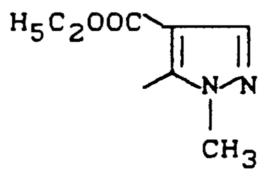 | O | O | O |
| $H_3C-C\equiv C-CH_2-$ | H | 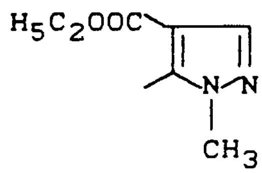 | O | O | O |
| 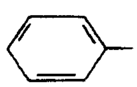 | H | 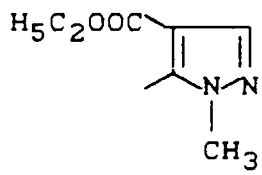 | O | O | O |
| 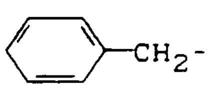 | H | 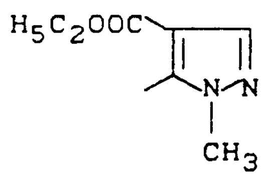 | O | O | O |
| 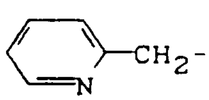 | H | 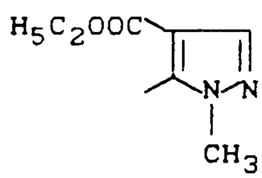 | O | O | O |
| 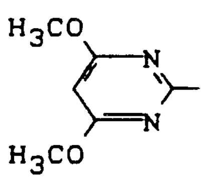 | H | 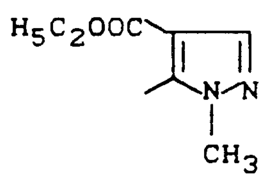 | O | O | O |

Die Verbindungen der Formel (I) sind bekannt und/oder können nach an sich bekannten Verfahren hergestellt werden (vergleiche EP-A 101 407; EP-A 127 469; EP-A 269 141; JP-A 82-140763 - zitiert in Chem. Abstracts 98, 53400k; JP-A 82-203059 - zitiert in Chem. Abstracts 99, 38221e; JP-A 85-214785 - zitiert in Chem. Abstracts 104, 109685u; US-P 4168152; DE-OS 26 44 504).

Gegenstand der vorliegenden Erfindung sind weiterhin neue sulfonylierte Carbamidsäureester der allgemeinen Formel (Ia)

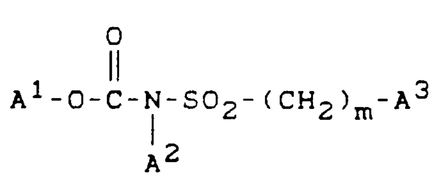

(Ia)

in welcher

m für die Zahlen 0, 1 oder 2 steht,

$A^1$ für jeweils gegebenenfalls substituiertes Alkenyl, Alkinyl, Cycloalkyl, Cycloalkylalkyl, Aryl, Aralkyl, Heterocylyl oder Heterocyclylalkyl steht,

$A^2$ für Wasserstoff oder für jeweils gegebenenfalls substituiertes Alkyl, Alkenyl, Alkinyl, Cycloalkyl, Cycloalkylalkyl, Aryl, Arylalkyl, Heterocyclyl, Heterocyclylalkyl, für ein Metalläquivalent, für Ammonium, Alkylammonium, Dialkylammonium oder Trialkylammonium steht oder zusammen mit $R^1$ für gegebenenfalls substituiertes Alkandiyl steht, und

$A^3$ für mindestens einmal in ortho-Position substituiertes Phenyl oder für jeweils gegebenenfalls substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht,

mit der Maßgabe, daß die Verbindungen ausgenommen sind, in denen

(1) $A^3$ für 2,4,6-Trimethyl-phenyl oder für gegebenenfalls zusätzlich substituiertes 2-Nitro-phenyl steht und m für die Zahl 0 steht, und

(2) $A^3$ für 2-Chlor-phenyl oder für 2-Methoxy-carbonylphenyl steht, $A^1$ für Allyl und gleichzeitig m für die Zahl 0 steht.

Die Verbindungen der Formel (Ia) werden von den Restedefinitionen der Formel (I) mitumfaßt.

Man erhält die neuen Verbindungen der Formel (Ia), wenn man

(a) Isocyanate der allgemeinen Formel (II)

$$O=C=N\text{-}SO_2\text{-}(CH_2)_m\text{-}A^3 \quad \text{(II)}$$

in welcher $A^3$ und m die oben angegebene Bedeutung haben,
mit Hydroxyverbindungen der allgemeinen Formel (III)

$$A^1\text{-}OH \quad \text{(III)}$$

in welcher

$A^1$ die oben angegebene Bedeutung hat,

gegebenenfalls in Gegenwart eines Verdünnungsmittels und gegebenenfalls in Gegenwart eines Reaktionshilfmittels umsetzt, oder wenn man

(b) Sulfonsäureamide der allgemeinen Formel (IV)

$$H_2N\text{-}SO_2\text{-}(CH_2)_m\text{-}A^3 \quad \text{(IV)}$$

in welcher

$A^3$ und m die oben angegebene Bedeutung haben,

mit Kohlensäurederivaten der allgemeinen Formel (V)

$$A^1\text{-}O\text{-}C(=O)\text{-}E^1 \quad \text{(V)}$$

in welcher

$A^1$ die oben angegebene Bedeutung hat und

$E^1$ für eine elektronenziehende Abgangsgruppe steht,

gegebenenfalls in Gegenwart eines Verdünnungsmittels und gegebenenfalls in Gegenwart eines Reaktionshilfsmittels umsetzt, oder wenn man

(c) Sulfonylverbindungen der allgemeinen Formel (VI)

$$E^2\text{-}SO_2\text{-}(CH_2)_m\text{-}A^3 \quad \text{(VI)}$$

in welcher

$A^3$ und m die oben angegebene Bedeutung haben und

$E^2$ für eine elektronenziehende Abgangsgruppe steht,

mit Carbamidsäureestern der allgemeinen Formel (VII)

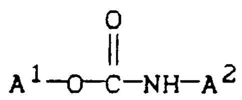 (VII)

in welcher

$A^1$ und $A^2$ die oben angegebene Bedeutung haben,

gegebenenfalls in Gegenwart eines Verdünnungsmittels und gegebenenfalls in Gegenwart eines Reaktionshilfsmittels umsetzt, oder wenn man

(d) sulfonylierte Carbamidsäureester der allgemeinen Formel (Ib)

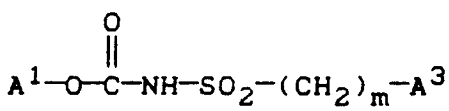 (Ib)

in welcher

$A^1$, $A^3$ und m die oben angegebene Bedeutung haben,

mit Alkylierungsmitteln der allgemeinen Formel (VIII)

$E^3$-$A^2$ (VIII)

in welcher

$A^2$ die oben angegebene Bedeutung hat und

$E^3$ für eine elektronenziehende Gruppe steht,

gegebenenfalls in Gegenwart eines Verdünnungsmittels und gegebenenfalls in Gegenwart eines Reaktionshilfsmittels umsetzt.

Auf prinzipiell gleiche Weise können auch die bekannten Verbindungen der allgemeinen Formel (I) hergestellt werden.

Bevorzugt werden die neuen sulfonylierten Carbamidsäureester der Formel (Ia), in welcher

m für die Zahlen 0, 1 oder 2 steht,

$A^1$ für Alkenyl mit 2 bis 8 Kohlenstoffatomen, Alkinyl mit 2 bis 8 Kohlenstoffatomen, Halogenalkenyl mit 2 bis 8 Kohlenstoffatomen und 1 bis 15 gleichen oder verschiedenen Halogenatomen, oder Halogenalkinyl mit 2 bis 8 Kohlenstoffatomen und 1 bis 13 gleichen oder verschiedenen Halogenatomen steht, oder $A^1$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen, Alkoxycarbonyl mit bis zu 4 Kohlenstoffatomen, oder $A^1$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $A^1$ für jeweils gegebenenfalls einfach bis vierfach, gleich oder verschieden substituiertes Heterocyclyl oder Heterocyclylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, oder Halogenalkyl, Halogenalkoxy oder Halogenalkylthio mit jeweils 1 bis 4 Kohlenstoffatomen und 1 bis 9 gleichen oder verschiedenen Halogenatomen,

$A^2$ für Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen

und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, oder $A^2$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen und Alkoxycarbonyl mit bis zu 4 Kohlenstoffatomen, oder $A^2$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $A^2$ für jeweils gegebenenfalls einfach bis vierfach, gleich oder verschieden substituiertes Heterocyclyl oder Heterocyclylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und 1 bis 9 gleichen oder verschiedenen Halogenatomen, oder

$A^2$ für ein Alkalimetall- oder ein Erdalkalimetall-Äquivalent, für Ammonium, $C_1$-$C_4$-Alkylammonium, Di-($C_1$-$C_4$-Alkyl)-ammonium oder Tri-($C_1$-$C_4$-Alkyl)-ammonium steht oder zusammen mit $A^1$ für gegebenenfalls durch Methyl oder Ethyl substituiertes geradkettiges Alkandiyl mit 2 bis 5 Kohlenstoffatomen steht, und

$A^3$ für mindestens einmal in ortho-Position und insgesamt einfach bis dreifach substituiertes Phenyl oder für jeweils mindestens einfach und gegebenenfalls bis dreifach substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht, wobei jeweils als Substituenten vorzugsweise ausgewählt sind: Halogen, Cyano, Alkyl, Halogenalkyl, Alkoxyalkyl, Alkoxy, Halogenalkoxy, Alkoxyalkoxy, Alkylthio, Halogenalkylthio, Alkylsulfinyl, Halogenalkylsulfinyl, Alkylsulfonyl oder Halogenalkylsulfonyl mit jeweils 1 bis 4 Kohlenstoffatomen in den Alkylteilen und gegebenenfalls 1 bis 9 Halogenatomen, Alkenyl, Halogenalkenyl, Alkinyl oder Halogenalkinyl mit jeweils 2 bis 4 Kohlenstoffatomen und gegebenenfalls 1 bis 7 Halogenatomen, Dialkylaminosulfonyl mit 1 oder 2 Kohlenstoffatomen in den Alkylteilen, Phenyl, Alkoxycarbonyl oder Halogenalkoxycarbonyl mit jeweils 1 bis 5 Kohlenstoffatomen und gegebenenfalls 1 bis 3 Halogenatomen im Alkoxyteil, sowie Dialkylaminocarbonyl mit 1 oder 2 Kohlenstoffatomen in den Alkylteilen,

mit der Maßgabe, daß die Verbindungen ausgenommen sind, in denen

(1) $A^3$ für 2,4,6-Trimethyl-phenyl und m für die Zahl 0 steht, und

(2) $A^3$ für 2-Chlor-phenyl oder für 2-Methoxycarbonylphenyl, $A^1$ für Allyl und m für die Zahl 0 steht.

Besonders bevorzugt werden die neuen Verbindungen der Formel (Ia), in welcher

m für die Zahlen 0 oder 1 steht,

$A^1$ für Allyl, Propargyl, für jeweils geradkettiges oder verzweigtes Butenyl, Pentenyl, Hexenyl, Butinyl, Pentinyl, Hexinyl oder Heptinyl, für Halogenalkenyl oder Halogenalkinyl mit 3 oder 4 Kohlenstoffatomen und 1 bis 3 Halogenatomen, insbesondere Fluor- und/oder Chloratomen, für jeweils gegebenenfalls einfach bis dreifach, gleich oder verschieden durch Fluor, Chlor, Brom, Methyl, Ethyl, n-Propyl, iso-Propyl, Ethenyl, Propenyl, Ethinyl, Propinyl, Methoxycarbonyl, Ethoxycarbonyl und Cyano substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls einfach bis dreifach gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl, oder für jeweils gegebenenfalls einfach oder zweifach, gleich oder verschieden durch Fluor, Chlor, Brom, Methyl, Ethyl, Trifluormethyl, Methoxy, Ethoxy und Difluormethoxy substituiertes Furyl, Furylmethyl, Thienyl, Thienylmethyl, Oxazolyl, Oxazolylmethyl, Isoxazolyl, Isoxazolylmethyl, Thiazolyl, Thiazolylmethyl, Isothiazolyl, Isothiazolylmethyl, Oxadiazolyl, Thiadiazolyl, Pyridinyl, Pyrimidinyl, Oxadiazolylmethyl, Thiadiazolylmethyl, Pyridinylmethyl, Pyrimidinylmethyl, Tetrahydrofuranylmethyl oder

Perhydrofuranylmethyl steht,

$A^2$ für Wasserstoff, Methyl, Ethyl, n-Propyl, Isopropyl, n-Butyl, Isobutyl, Allyl, Propargyl, für jeweils geradkettiges oder verzweigtes Pentyl, Hexyl, Butenyl, Pentenyl, Hexenyl, Butinyl, Pentinyl, Hexinyl oder Heptinyl, für Halogenalkyl, Halogenalkenyl oder Halogenalkinyl mit bis zu 5 Kohlenstoffatomen, letztere mit insbesondere 3 oder 4 Kohlenstoffatomen, und jeweils 1 bis 3 Halogenatomen, insbesondere Fluor- und/oder Chloratomen, für Cyanalkyl, Alkoxyalkyl oder Alkoxycarbonylalkyl mit jeweils 1 bis 4 Kohlenstoffatomen in den einzelnen Alkylteilen, für jeweils gegebenenfalls einfach bis dreifach, gleich oder verschieden durch Fluor, Chlor, Brom, Methyl, Ethyl, n-Propyl, iso-Propyl, Ethenyl, Propenyl, Ethinyl, Propinyl, Methoxycarbonyl, Ethoxycarbonyl und Cyano substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls einfach bis dreifach, gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl steht, oder für Natrium, Kalium, Ammonium, Methylammonium, Ethylammonium, n-Propylammonium, Isopropylammonium, Dimethylammonium, Diethylammonium, Dipropylammonium, Diisopropylammonium, Trimethylammonium, Triethylammonium oder Tripropylammonium steht, oder zusammen mit $A^1$ für Dimethylen ($-CH_2CH_2-$) oder Trimethylen ($-CH_2CH_2CH_2-$) steht, und

$A^3$ für in ortho-Position durch Fluor, Chlor, Brom, Cyano, Methyl, Ethyl, Trifluormethyl, Methoxy, Ethoxy, Difluormethoxy, Trifluormethoxy, Fluorethoxy, Chlorethoxy, Methoxyethoxy, Ethoxyethoxy, Methylthio, Ethylthio, Methylsulfinyl, Ethylsulfinyl, Methylsulfonyl, Ethylsulfonyl, Dimethylaminosulfonyl, Phenyl, Methoxycarbonyl, Ethoxycarbonyl, Propoxycarbonyl, Isopropoxycarbonyl, Butoxycarbonyl, Neopentyloxycarbonyl, Chlorethoxycarbonyl oder Dimethylaminocarbonyl substituiertes Phenyl steht, welches gegebenenfalls einen weiteren Fluor-, Chlor- oder Brom-Substituenten enthält, oder für Pyridinyl steht, welches durch Fluor, Chlor, Brom, Methoxycarbonyl, Ethoxycarbonyl, Dimethylaminocarbonyl, Methylsulfonyl oder Ethylsulfonyl substituiert ist, oder für Thienyl steht, welches durch Chlor, Brom, Methoxycarbonyl oder Ethoxycarbonyl substituiert ist, oder für Pyrazolyl steht, welches durch Chlor, Brom, Methyl, Ethyl, Phenyl, Methoxycarbonyl und/oder Ethoxycarbonyl substituiert ist,

wobei die Verbindungen ausgenommen sind, in denen $A^3$ für 2-Chlor-phenyl oder für 2-Methoxycarbonylphenyl und $A^1$ für Allyl steht.

Ganz besonders bevorzugt werden die neuen Verbindungen der Formel (I), in welcher

m für die Zahlen 0 oder 1 steht,

$A^1$ die oben als besonders bevorzugt angegebene Bedeutung hat,

$A^2$ für Wasserstoff, Methyl, Ethyl, n-Propyl, Isopropyl, Allyl, Propargyl oder Methylpropargyl steht und

$A^3$ die oben als besonders bevorzugt angegebene Bedeutung hat,

wobei die Verbindungen ausgenommen sind, in denen $A^3$ für 2-Chlor-phenyl oder für 2-Methoxycarbonylphenyl und $A^1$ für Allyl steht.

Verwendet man für das erfindungsgemäße Verfahren (a) beispielsweise 2-Methoxycarbonyl-phenylmethylsulfonylisocyanat und Propargylalkohol als Ausgangsstoffe, so kann der Reaktionsablauf durch das folgende Formelschema wiedergegeben werden:

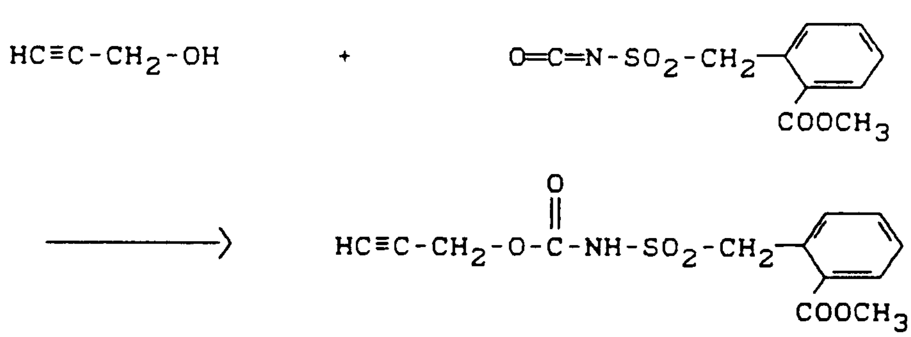

Verwendet man für das erfindungsgemäße Verfahren (b) beispielsweise 2-Trifluormethyl-benzolsulfonamid und Chlorameisensäure-phenylester als Ausgangsstoffe, so kann der Reaktionsablauf durch das folgende Formelschema wiedergegeben werden:

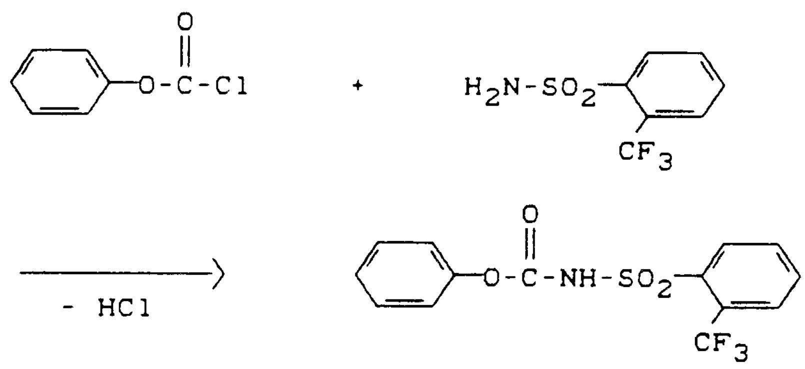


Verwendet man für das erfindungsgemäße Verfahren (c) beispielsweise 2-Chlor-benzolsulfonsäurechlorid und 2-Oxazolidinon als Ausgangsstoffe, so kann der Reaktionsablauf durch das folgende Formelschema wiedergegeben werden:

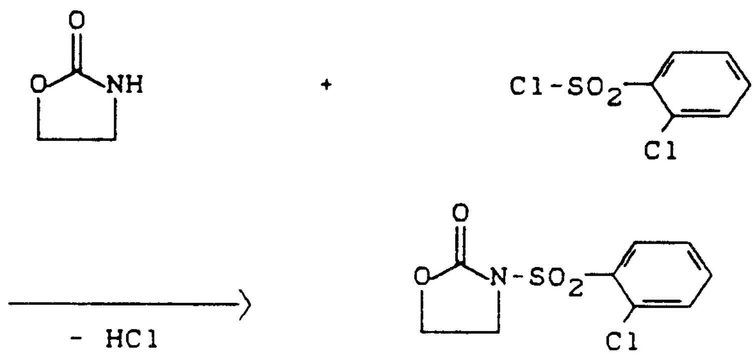


Verwendet man für das erfindungsgemäße Verfahren (d) beispielsweise N-(2-Fluor-phenylsulfonyl)-O-benzyl-carbamidsäureester und Methyliodid als Ausgangsstoffe, so kann der Reaktionsablauf durch das folgende Formelschema wiedergegeben werden:

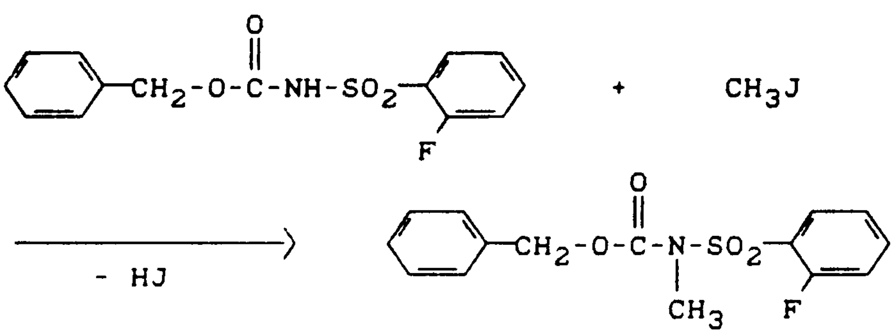


In Formel (II) haben $A^3$ und m vorzugsweise bzw. insbesondere diejenigen Bedeutungen, die bereits oben im Zusammenhang mit der Beschreibung der erfindungsgemäßen Verbindungen der Formel (Ia) vorzugsweise bzw. als insbesondere bevorzugt für $A^3$ und m angegeben wurden.

Die Isocyanate der Formel (II) sind bekannt und/oder können nach an sich bekannten Verfahren hergestellt werden (vergleiche US-P 4127405; US-P 4169719; US-P 4371391; EP-A 7687; EP-A 13480; EP-A 21641; EP-A 23141; EP-A 23422; EP-A 30139; EP-A 35893; EP-A 44808; EP-A 44809; EP-A 48143; EP-A 51466; EP-A 64322; EP-A 70041; EP-A 173312).

In Formel (III) hat $A^1$ vorzugsweise bzw. insbesondere diejenige Bedeutung, die bereits oben im Zusammenhang mit der Beschreibung der erfindungsgemäßen Verbindungen der Formel (Ia) vorzugsweise bzw. als insbesondere bevorzugt für $A^1$ angegeben wurde.

Die Hydroxyverbindungen der Formel (III) sind bekannte Synthesechemikalien.

In Formel (IV) haben $A^3$ und m vorzugsweise bzw. insbesondere diejenigen Bedeutungen, die bereits

oben im Zusammenhang mit der Beschreibung der erfindungsgemäßen Verbindungen der Formel (Ia) vorzugsweise bzw. als insbesondere bevorzugt für $A^3$ und m angegeben wurden.

Die Sulfonsäureamide der Formel (IV) sind bekannt und/oder können nach an sich bekannten Verfahren hergestellt werden (vergleiche US-P 4127405; US-P 4169719; US-P 4371391; EP-A 7 687; EP-A 13 480; EP-A 21 641; EP-A 23 141; EP-A 23 422; EP-A 30 139; EP-A 35 893; EP-A 44 808; EP-A 44 809; EP-A 48 143; EP-A 51 466; EP-A 64 322; EP-A 70 041; EP-A 173 312).

In Formel (V) hat $A^1$ vorzugsweise bzw. insbesondere diejenige Bedeutung, die bereits oben im Zusammenhang mit der Beschreibung der erfindungsgemäßen Verbindungen der Formel (Ia) vorzugsweise bzw. als insbesondere bevorzugt für $A^1$ angegeben wurde und $E^1$ steht vorzugsweise für Halogen, Methoxy, Ethoxy, Benzyloxy oder Phenoxy, insbesondere für Chlor.

Die Kohlensäurederivate der Formel (V) sind weitgehend bekannte Synthesechemikalien.

In Formel (VI) haben $A^3$ und m vorzugsweise bzw. insbesondere diejenigen Bedeutungen, die bereits oben im Zusammenhang mit der Beschreibung der erfindungsgemäßen Verbindungen der Formel (Ia) vorzugsweise bzw. als insbesondere bevorzugt für $A^3$ und m angegeben wurden und $E^2$ steht vorzugsweise für Halogen, insbesondere für Chlor.

Die Sulfonylverbindungen der Formel (VI) sind bekannt und/oder können nach an sich bekannten Verfahren hergestellt werden (vergleiche J. Org. Chem. 25 (1960), 1824; loc. cit. 33 (1968), 2104; DE-AS 23 08 262; EP-A 23 140; EP-A 23 141; EP-A 23 422; EP-A 35 893; EP-A 48 143; EP-A 51 466; EP-A 64 322; EP-A 70 041; EP-A 44 808; EP-A 44 809; US-P 29 29 820; US-P 42 82 242; US-P 43 48 220; US-P 43 72 778).

In Formel (VII) haben $A^1$ und $A^2$ vorzugsweise bzw. insbesondere diejenigen Bedeutungen, die bereits oben im Zusammenhang mit der Beschreibung der erfindungsgemäßen Verbindungen der Formel (Ia) vorzugsweise bzw. als insbesondere bevorzugt für $A^1$ und $A^2$ angegeben wurden.

Die Carbamidsäureester der Formel (VII) sind weitgehend bekannte Synthesechemikalien.

In Formel (Ib) haben $A^1$, $A^3$ und m vorzugsweise bzw. insbesondere diejenigen Bedeutungen, die bereits oben im Zusammenhang mit der Beschreibung der erfindungsgemäßen Verbindungen der Formel (Ia) vorzugsweise bzw. als insbesondere bevorzugt für $A^1$, $A^3$ und m angegeben wurden.

Die sulfonylierten Carbamidsäureester der Formel (Ib) sind erfindungsgemäße, neue Verbindungen; sie können nach den erfindungsgemäßen Verfahren (a), (b) oder (c) hergestellt werden.

In Formel (VIII) hat $A^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $A^2$ angegeben wurde und $E^3$ steht vorzugsweise für Halogen, Methylsulfonyloxy, Methoxysulfonyloxy, Ethoxysulfonyloxy, Phenylsulfonyloxy oder p-Tolylsulfonyloxy, insbesondere für Chlor, Brom oder Iod.

Die Alkylierungsmittel der Formel (VIII) sind weitgehend bekannte Synthesechemikalien.

Die erfindungsgemäßen Verfahren (a), (b), (c) und (d) zur Herstellung der neuen Verbindungen der Formel (Ia) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die erfindungsgemäßen Verfahren (a), (b), (c) und (d) werden gegebenenfalls unter Verwendung von Reaktionshilfsmitteln durchgeführt. Geeignete Reaktionshilfmittel sind hierbei jeweils vor allem basische Verbindungen. Hierzu gehören beispielsweise Alkalimetall- und Erdalkalimetallhydride, wie Lithium-, Natrium-, Kalium- und Calciumhydrid, Alkalimetall- und Erdalkalimetallhydroxide, wie Lithium-, Natrium-, Kalium- und Calciumhydroxid, Alkalimetall- und Erdalkalimetallcarbonate oder -hydrogencarbonate, wie Natrium- und Kaliumcarbonat oder -hydrogencarbonat sowie Calciumcarbonat, Alkalimetallalkoholate, wie Kalium-tert-butylat, sowie basische Stickstoffverbindungen, wie Trimethylamin, Triethylamin, Ethyl-diisopropylamin, Dicyclohexylamin, N,N-Dimethylbenzylamin, Pyridin, 1,5-Diazabicyclo-[4,3,0]-non-5-en (DBN), 1,8-Diazabicyclo-[5,4,0]-undec-7-en (DBU) und 1,8-Diazabicyclo-[2,2,2]-octan (DABCO).

Die Reaktionstemperaturen können bei den erfindungsgemäßen Verfahren (a), (b), (c) und (d) in einem größeren Bereich variiert werden. Im allgemeinen arbeitet man bei Temperaturen zwischen -20 °C und +150 °C, vorzugsweise bei Temperaturen zwischen 0 °C und 100 °C.

Die erfindungsgemäßen Verfahren (a), (b), (c) und (d) werden im allgemeinen bei atmosphärischem

Druck ("Normaldruck") durchgeführt. Es ist jedoch auch möglich, unter erhöhtem oder vermindertem Druck zu arbeiten.

Zur Durchführung der erfindungsgemäßen Verfahren (a), (b), (c) und (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden, Die Reaktionen werden im allgemeinen in einem geeigneten Verdünnungsmittel gegebenenfalls in Gegenwart eines Reaktionshilfsmittels durchgeführt, und das Reaktionsgemisch wird mehrere Stunden bei der jeweils erforderlichen Temperatur gerührt. Die Aufarbeitung erfolgt jeweils nach üblichen Methoden (vergleiche die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäß zu verwendenden Verbindungen der Formel (I) eignen sich zur selektiven Bekämpfung von monokotylen und dikotylen Unkräutern in monokotylen und dikotylen Kulturen, wie z.B. in Weizen, Gerste, Mais und Soja, sowohl im Vorauflauf- als auch im Nachauflauf-Verfahren.

In gewissem Umfang zeigen sie auch insektizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische

Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und

fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl-thio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid(BUTACHLOR); 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[-(1H)-pyrazol-1-yl-methyl]-acetamid (METAZACHLOR); 2-Ethyl-6-methyl-N-(1-methyl-2-methoxyethyl)-chlo-

racetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitrophenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 2-Chlor-N-isopropylacetanilid (PROPACHLOR); Isopropyl-N-phenyl-carbamat (PROPHAM); 0-(6-Chlor-3-phenyl-pyridazin-4-yl)-S-octylthiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino)-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Anwendungsbeispiele:

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen beispielsweise die Verbindungen gemäß den Herstellungsbeispielen 2, 7, 10 und 11 bei guter Verträglichkeit gegenüber Kulturpflanzen, wie Gerste, Weizen und Mais, starke Wirkung gegen Unkräuter.

Beispiel B

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 7, 10 und 29 bei guter Verträglichkeit gegenüber Kulturpflanzen, wie Gerste, Weizen, Mais und Soja, starke Wirkung gegen Unkräuter.

Herstellungsbeispiele:

Beispiel 1

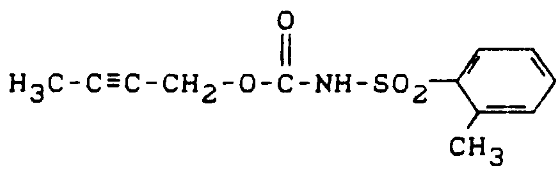

(Verfahren (a))

Eine Lösung von 1,40 g (0,02 Mol) 2-Butin-1-ol in 7 ml Methylenchlorid wird bei 20° C zu einer Lösung von 3,94 g (0,02 Mol) 2-Methyl-phenylsulfonylisocyanat in 14 ml Methylenchlorid gegeben und das Reaktionsgemisch wird etwa eine Stunde gerührt. Dann wird das Lösungsmittel im Wasserstrahlvakuum sorgfältig abdestilliert.

Man erhält 4,83 g (90 % der Theorie) N-(2-Methyl-phenylsulfonyl)-O-(2-butin-1-yl)-carbamidsäureester als festen Rückstand, welcher bei 99° C bis 105° C schmilzt.

$^1$H-NMR (80 MHz, $CDCl_3$, δ): 8,16 (dd, 1H, J = 8,0 Hz, J' = 1,0 Hz); 7,89 (bs, 1H); 7,53 (dt, 1H, J = 8,0Hz, J' = 1,0 Hz); 7,37 (t, 1H, J = 8,0 Hz); 7,33 (d, 1H, J = 8,0 Hz); 4,61 (q, 2H, J = 1 Hz); 2,67 (s, 3H); 1,81 (t, 3H, J = 1 Hz).

Beispiel 2

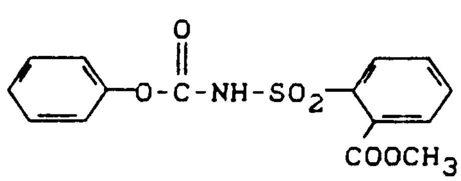

(Verfahren (a))

Eine Mischung aus 2,41 g (0,01 Mol) 2-Methoxycarbonylphenylsulfonylisocyanat, 0,94 g (0,01 Mol) Phenol und 8 ml Methylenchlorid wird etwa eine Stunde bei 20° C gerührt. Dann wird das Lösungsmittel im Wasserstrahlvakuum sorgfältig abdestilliert.

Man erhält 3,28 g (98 % der Theorie) N-(2-Methoxycarbonyl-phenylsulfonyl)-O-phenyl-carbamidsäureester als öligen Rückstand.

$^1H$-NMR (80 MHz, $CDCl_3$, δ): 8,74 (bs, 1H); 8,29 (dd, 1H, J = 8,0 Hz, J' = 1 Hz); 7,82 (dd, 1H, J = 8,0 Hz, J' = 1 Hz); 7,65 (m, 2H); 7,29 (t, 2H, J = 8,2 Hz); 7,18 (t, 1H, J = 8,2 Hz); 7,03 (d, 2H, J = 8,2 Hz); 3,99 (s, 3H).

Beispiel 3

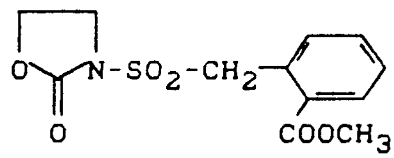

(Verfahren (c))

4,69 g (0,019 Mol) 2-Methoxycarbonyl-phenylmethansulfonsäurechlorid werden zu einer Mischung aus 1,74 g (0,02 Mol) 2-Oxazolidinon, 2,02 g (0,02 Mol) Triethylamin und 10 ml Methylenchorid gegeben und das Reaktionsgemisch wird 5 Stunden bei 20 °C gerührt. Dann wird mit Wasser auf etwa das doppelte Volumen verdünnt, nach Durchschütteln die organische Phase abgetrennt, mit Natriumsulfat getrocknet und filtriert. Nach Einengen des Filtrats wird der Rückstand durch Säulenchromatographie (Kieselgel; Petrolether/Ethylacetat) gereinigt.

Man erhält 1,51 g (27 % der Theorie) N-(2-Methoxycarbonyl-phenylmethylsulfonyl)-oxazolidin-2-on als festes Produkt, welches bei 84 °C bis 90 °C schmilzt.

$^1H$-NMR (80 MHz, $CDCl_3$, δ): 8,00 (dd, 1H, J = 8,0 Hz, J' = 1 Hz); 7,65-7,45 (m, 3H); 5,35 (s, 2H); 4,29 (t, 2H, J = 8,2 Hz); 3,95 (s, 3H); 3,61 (t, 2H, J = 8,2 Hz).

Beispiel 4

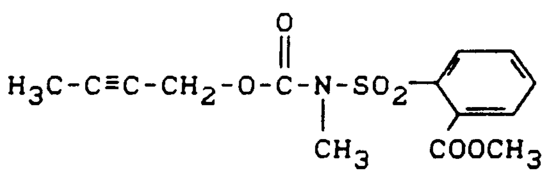

(Verfahren (d))

Eine Mischung aus 1,66 g (5 mMol) N-(2-Methoxycarbonylphenylsulfonyl)-O-(2-butin-1-yl)-carbamidsäureester, 1,04 g (7,5 mMol) Kaliumcarbonat und 15 ml Acetonitril wird 2 Stunden unter Rückfluß erhitzt. Nach Abkühlen auf 20 °C werden 1,42 g (10 mMol) Methyliodid dazugegeben und das Reaktionsgemisch wird weitere 8 Stunden unter Rückfluß erhitzt.

Nach Einengen wird der Rückstand mit Wasser/Methylenchlorid geschüttelt, die organische Phase abgetrennt, mit Natriumsulfat getrocknet und filtriert. Vom Filtrat wird das Lösungsmittel im Wasserstrahlvakuum sorgfältig abdestilliert.

Man erhält 1,31 g (80,5 % der Theorie) N-Methyl-N-(2-methoxycarbonyl-phenylsulfonyl)-O-(2-butin-1-yl)-carbamidsäureester als festen Rückstand, welcher bei 74 °C bis 78 °C schmilzt.

$^1H$-NMR (80 MHz, $CDCl_3$, δ): 8,26 (m, 1H); 7,65-7,50 (m, 3H); 4,57 (q, 2H, J = 1 Hz); 3,88 (s, 3H); 3,31 (s, 3H); 1,74 (t, 3H, J = 1 Hz).

Analog zu den Herstellungsbeispielen 1 bis 4 und gemäß der allgemeinen Beschreibung der erfindungsgemäßen Herstellungsverfahren können beispielsweise auch die in der nachstehenden Tabelle 2 aufgeführten Verbindungen der Formel (I) - welche die Verbindungen der Formel (Ia) einschließt - erhalten werden.

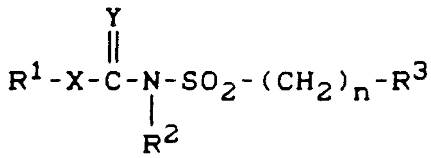

(I)

Tabelle 2: Herstellungsbeispiele für die Verbindungen der Formel (I)

| Bsp.-Nr. | $R^1$ | $R^2$ | $R^3$ | n | X | Y | Schmelzpunkt (°C) |
|---|---|---|---|---|---|---|---|
| 5 | 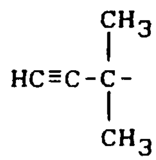 | H | 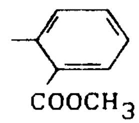 | O | O | O | 115-120 |
| 6 | 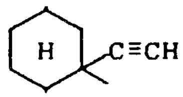 | H | 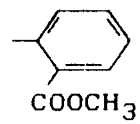 | O | O | O | Öl |
| 7 | $H_3C-C{\equiv}C-CH_2-$ | H | 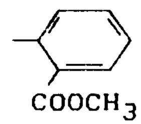 | O | O | O | 91-98 |
| 8 | $(CH_3)_3C-$ | H | 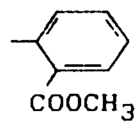 | O | O | O | 87-91 |
| 9 | 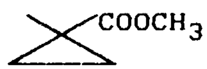 | H | 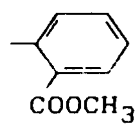 | O | O | O | 139-142 |
| 10 | 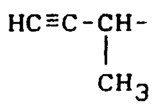 | H | 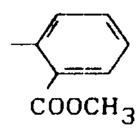 | O | O | O | 149-153 |

**Tabelle 2:** Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 11 | $HC{\equiv}C-CH_2-$ | H | 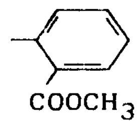 | 0 | O | O | 117-124 |
| 12 | $(CH_3)_2CH-$ | H | 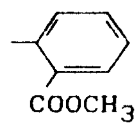 | 0 | O | O | 96-100 |
| 13 | $HC{\equiv}C-CH_2CH_2-$ | H | 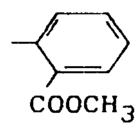 | 0 | O | O | amorph |
| 14 | 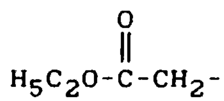 | H | 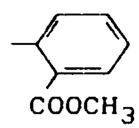 | 0 | S | O | 97-103 |
| 15 | 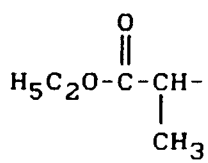 | H | 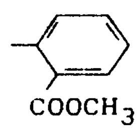 | 0 | O | O | Öl |
| 16 | $H_2C=CH-CH_2-$ | H | 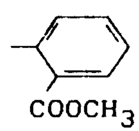 | 0 | O | O | 35 |

**Tabelle 2:** Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 17 | 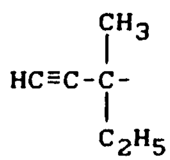 | H | 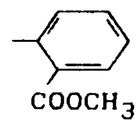 | 0 | O | O | 104-111 |
| 18 | 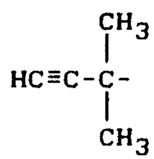 | H | 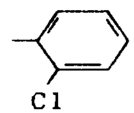 | 0 | O | O | 139-144 |
| 19 | $H_3C-C\equiv C-CH_2-$ | H | 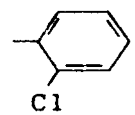 | 0 | O | O | 113 |
| 20 | $HC\equiv C-CH_2CH_2-$ | H | 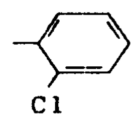 | 0 | O | O | 73 |
| 21 | 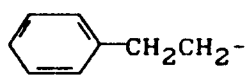 | H | 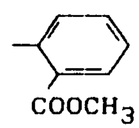 | 0 | O | O | amorph |
| 22 | 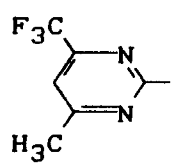 | H | 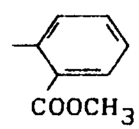 | 0 | S | O | 115 |

**Tabelle 2:** Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 23 | $-CH_2CH_2-$ | | 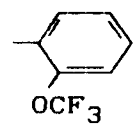 | 0 | 0 | 0 | 126 |
| 24 | 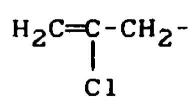 | H | 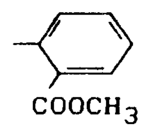 | 0 | 0 | 0 | Öl |
| 25 | 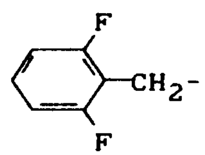 | H | 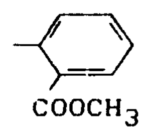 | 0 | 0 | 0 | 121 |
| 26 | $-CH_2CH_2-$ | | 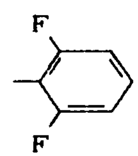 | 0 | 0 | 0 | 138 |
| 27 | $H_3C-C\equiv C-CH_2-$ | H | 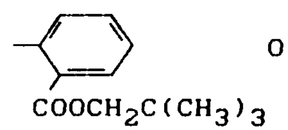 | 0 | 0 | 0 | 102 |
| 28 | $NC-CH_2CH_2-$ | H | 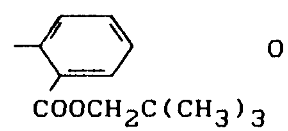 | 0 | 0 | 0 | 108 |

**Tabelle 2:** Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 29 | $H_3C-C≡C-CH_2-$ | H | 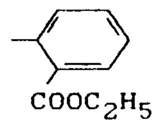 | 0 | O | O | 118-121 |
| 30 | $H_5C_2-C≡C-CH_2CH_2-$ | H | 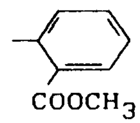 | 0 | O | O | Öl |
| 31 | $Cl_2C=C-CH-$<br>Cl $CH_3$ | H | 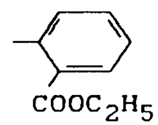 | 0 | O | O | 104-107 |
| 32 | $H_3C-(CH_2)_3-C≡C-CH_2-$ | H | 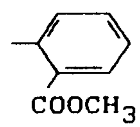 | 0 | O | O | Öl |
| 33 | $H_3C-(CH_2)_2-C≡C-CH_2-$ | H | 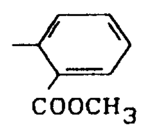 | 0 | O | O | 97-101 |
| 34 | $H_5C_2-$ | H | 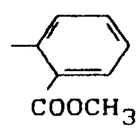 | 0 | S | O | 78-86 |

<u>Tabelle 2:</u> Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 35 | $Cl-CH_2CH_2-$ | H | 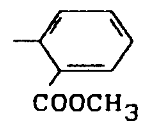 | 0 | O | O | 86-93 |
| 36 | $F_3C-CH_2-$ | H | 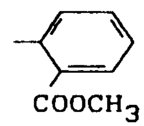 | 0 | O | O | Öl |
| 37 | 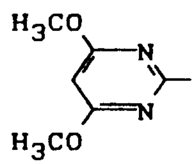 | H | 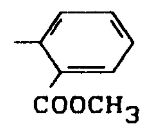 | 0 | O | O | 104-106 |
| 38 | 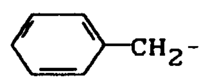 | H | 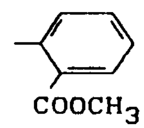 | 0 | O | O | 106 |
| 39 | $H_5C_2OCH_2CH_2-$ | H | 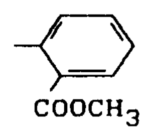 | 0 | O | O | 71-74 |
| 40 | 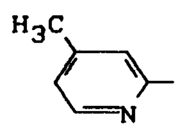 | H | 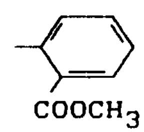 | 0 | O | O | Öl |

<u>Tabelle 2:</u> Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 41 | 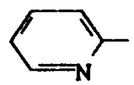 | H | 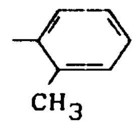 | 0 | O | O | Öl |
| 42 | $H_3C-$ | H | 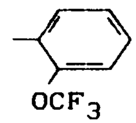 | 0 | O | O | 151-155 |
| 43 | $H_3C-C{\equiv}C-CH_2-$ | H | 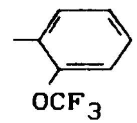 | 0 | O | O | 106-109 |
| 44 | 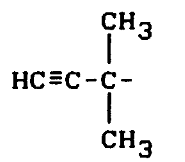 | H | 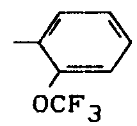 | 0 | O | O | 101-106 |
| 45 | $H_3C-C{\equiv}C-CH_2-$ | H | 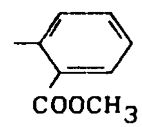 | 1 | O | O | Öl |
| 46 | $H_3C-$ | H | 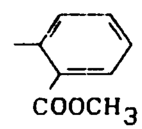 | 1 | O | O | Öl |

**Tabelle 2:** Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 47 | $Cl-CH_2-C{\equiv}C-CH_2-$ | H | 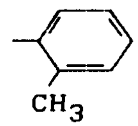 | 0 | O | O | 91 |
| 48 | $Cl-CH_2-C{\equiv}C-CH_2-$ | H | 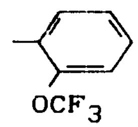 | 0 | O | O | 103-107 |
| 49 | $Cl-CH_2-C{\equiv}C-CH_2-$ | H | 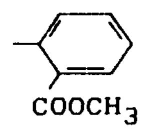 | 0 | O | O | 101 |
| 50 | 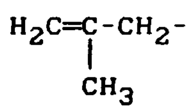 | H | 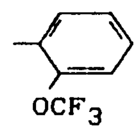 | 0 | O | O | Öl |
| 51 | 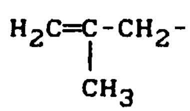 | H | 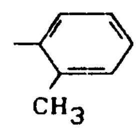 | 0 | O | O | Öl |
| 52 | $H_3C-CH{=}CH-CH_2-$ | H | 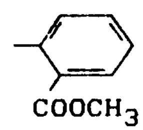 | 0 | O | O | Öl |

**Tabelle 2:** Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 53 | $H_3C-(CH_2)_2-CH=CH-CH_2-$ | H | 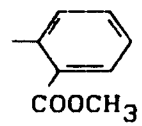 | 0 | O | O | Öl |
| 54 | $H_3C-C\equiv C-CH_2-$ | $CH(CH_3)_2$ | 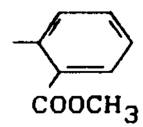 | 0 | O | O | Öl |
| 55 | 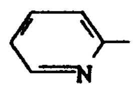 | H | 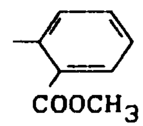 | 0 | O | O | amorph |
| 56 | 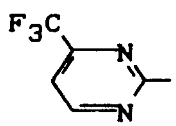 | H | 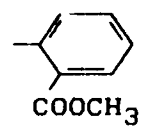 | 0 | S | O | amorph |
| 57 | 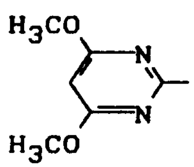 | H | 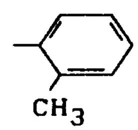 | 0 | O | O | Öl |

Tabelle 2: Herstellungsbeispiele für die Verbindungen der Formel (I) (Fortsetzung)

| Bsp.-Nr. | $R^1$ | $R^2$ | $R^3$ | n | X | Y | Schmelzpunkt (°C) |
|---|---|---|---|---|---|---|---|
| 58 | 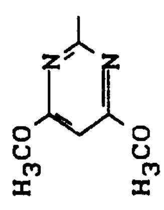 | H | 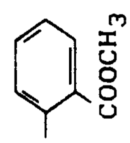 | 1 | O | O | Öl |

## Patentansprüche

1. Herbizide Mittel, gekennzeichnet durch einen Gehalt an mindestens einem sulfonylierten (Thio)-Carbamidsäureester der allgemeinen Formel (I)

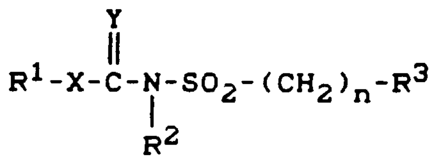

(I)

in welcher

n für die Zahlen 0, 1 oder 2 steht,

$R^1$ für jeweils gegebenenfalls substituiertes Alkyl, Alkenyl, Alkinyl, Cycloalkyl, Cycloalkylalkyl, Aryl, Arylalkyl, Heterocyclyl oder Heterocyclylalkyl steht,

$R^2$ für Wasserstoff oder für jeweils gegebenenfalls substituiertes Alkyl, Alkenyl, Alkinyl, Cycloalkyl, Cycloalkylalkyl, Aryl, Arylalkyl, Heterocyclyl, Heterocyclylalkyl, für ein Metalläquivalent, für Ammonium, Alkylammonium, Dialkylammonium oder Trialkylammonium steht oder zusammen mit $R^1$ für gegebenenfalls substituiertes Alkandiyl steht,

$R^3$ für mindestens einmal in ortho-Position substituiertes Phenyl oder für jeweils gegebenenfalls substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht,

X für Sauerstoff oder Schwefel steht und

Y für Sauerstoff oder Schwefel steht,

wobei die Verbindungen ausgenommen sind, in denen

(a) $R^3$ für 2-Nitro-phenyl steht, welches zusätzlich noch substituiert sein kann und

(b) $R^3$ für 2-Methyl-phenyl oder 2-Methoxycarbonylphenyl steht und $R^1$ für Methyl oder Ethyl steht, n für die Zahl 0, $R^2$ für Wasserstoff und X und Y für Sauerstoff stehen.

**2.** Herbizide Mittel gemäß Anspruch 1, dadurch gekennzeichnet, daß

n für die Zahlen 0, 1 oder 2 steht,

$R^1$ für Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatommen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, oder $R^1$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen, oder $R^1$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $R^1$ für jeweils gegebenenfalls einfach bis vierfach, gleich oder verschieden substituiertes Heterocyclyl oder Heterocylylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, oder Halogenalkyl, Halogenalkoxy oder Halogenalkylthio mit jeweils 1 bis 4 Kohlenstoffatomen und 1 bis 9 gleichen oder verschiedenen Halogenatomen,

$R^2$ für Wasserstoff, für Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, oder $R^2$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen, Alkoxycarbonyl mit bis zu 4 Kohlenstoffatomen, oder $R^2$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $R^2$ für jeweils gegebenenfalls einfach bis vierfach, gleich oder verschieden substituiertes Heterocyclyl oder Heterocyclylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und 1 bis 9 gleichen oder verschiedenen Halogenatomen, oder $R^2$ für ein Alkalimetall- oder ein Erdalkalimetall-Äquivalent, für Ammonium, $C_1$-$C_4$-Alkylammonium, Di-($C_1$-$C_4$-Alkyl)-ammonium oder Tri-($C_1$-$C_4$-Alkyl)-ammonium steht oder zusammen mit $R^1$ für gegebenenfalls durch Methyl oder Ethyl substituiertes geradkettiges Alkandiyl mit 2 bis 5 Kohlenstoffatomen steht,

$R^3$ für mindestens einmal in ortho-Position und insgesamt einfach bis dreifach substituiertes Phenyl oder für jeweils mindestens einfach und gegebenenfalls bis dreifach substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht, wobei jeweils als Substituenten ausgewählt sind: Halogen, Cyano, Alkyl, Halogenalkyl, Alkoxyalkyl, Alkoxy, Halogenalkoxy, Alkoxyalkoxy, Alkylthio, Halogenalkylthio, Alkylsulfinyl, Halogenalkylsulfinyl, Alkylsulfonyl oder Halogenalkylsulfonyl mit jeweils 1 bis 4 Kohlenstoffatomen in den Alkylteilen und gegebenenfalls 1 bis 9 Halogenatomen, Alkenyl, Halogenalkenyl, Alkinyl oder Halogenalkinyl mit jeweils 2 bis 4 Kohlenstoffatomen und gegebenenfalls 1 bis 7 Halogenatomen, Dialkylaminosulfonyl mit 1 oder 2 Kohlenstoffatomen in den Alkylteilen, Phenyl, Alkoxycarbonyl oder Halogenalkoxycarbonyl mit jeweils 1 bis 5 Kohlenstoffatomen und gegebenenfalls 1 bis 3 Halogenatomen im Alkoxyteil, sowie Dialkylaminocarbonyl mit 1 oder 2 Kohlenstoffatomen in den Alkylteilen,

X für Sauerstoff oder Schwefel steht und

Y für Sauerstoff oder Schwefel steht.

**3.** Herbizide Mittel gemäß Anspruch 1, dadurch gekennzeichnet, daß

n für die Zahlen 0 oder 1 steht,

$R^1$ für Methyl, Ethyl, n-Propyl, Isopropyl, n-Butyl, Isobutyl, sec-Butyl, tert-Butyl, Allyl, Propargyl, für jeweils geradkettiges oder verzweigtes Pentyl, Hexyl, Butenyl, Pentenyl, Hexenyl, Butinyl, Pentinyl, Hexinyl oder Heptinyl, für Halogenalkyl, Halogenalkenyl oder Halogenalkinyl mit bis zu 5 Kohlenstoffatomen und jeweils 1 bis 3 Halogenatomen, für Cyanalkyl, Alkoxyalkyl oder Alkoxycarbonylalkyl mit jeweils 1 bis 4 Kohlenstoffatomen in den einzelnen Alkylteilen, für jeweils gegebenenfalls einfach bis dreifach, gleich oder verschieden durch Fluor, Chlor, Brom, Cyano, Methyl, Ethyl, n- oder iso-Propyl, Ethenyl, Propenyl, Ethinyl, Propinyl, Methoxycarbonyl und Ethoxycarbonyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls einfach bis dreifach, gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl, oder für jeweils gegebenenfalls einfach oder zweifach, gleich oder verschieden durch Fluor, Chlor, Brom, Methyl, Ethyl, Trifluormethyl, Methoxy, Ethoxy und Difluormethoxy substituiertes Furyl, Furylmethyl, Thienyl, Thienylmethyl, Oxazolyl, Oxazolylmethyl, Isoxazolyl, Isoxazolylmethyl, Thiazolyl, Thiazolylmethyl, Isothiazolyl, Isothiazolylmethyl, Oxadiazolyl, Thiadiazolyl, Pyridinyl, Pyrimidinyl, Oxadiazolylmethyl, Thiadiazolylmethyl, Pyridinylmethyl, Pyrimidinylmethyl, Tetrahydrofuranylmethyl oder Perhydrofuranylmethyl steht,

$R^2$ für Wasserstoff, Methyl, Ethyl, n-Propyl, Isopropyl, n-Butyl, Isobutyl, Allyl, Propargyl, für jeweils geradkettiges oder verzweigtes Pentyl, Hexyl, Butenyl, Pentenyl, Hexenyl, Butinyl, Pentinyl, Hexinyl oder Heptinyl, für Halogenalkyl, Halogenalkenyl oder Halogenalkinyl mit bis zu 5 Kohlenstoffatomen

und jeweils 1 bis 3 Halogenatomen, für Cyanalkyl, Alkoxyalkyl oder Alkoxycarbonylalkyl mit jeweils 1 bis 4 Kohlenstoffatomen in den einzelnen Alkylteilen, für jeweils gegebenenfalls einfach bis dreifach, gleich oder verschieden durch Fluor, Chlor, Brom, Methyl, Ethyl, n-Propyl, Ethenyl, Propenyl, Ethinyl, Propinyl, Methoxycarbonyl, Ethoxycarbonyl und Cyano substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls einfach bis dreifach, gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl steht, oder für Natrium, Kalium, Ammonium, Methylammonium, Ethylammonium, n-Proylammonium, Isopropylammonium, Dimethylammonium, Diethylammonium, Di-n-propylammonium, Diisopropylammonium, Trimethylammonium, Triethylammonium oder Tri-n-propylammonium steht, oder zusammen mit $R^1$ für Dimethylen ($-CH_2CH_2-$) oder Trimethylen ($-CH_2CH_2CH_2-$) steht,

$R^3$ für in ortho-Position durch Fluor, Chlor, Brom, Cyano, Methyl, Ethyl, Trifluormethyl, Methoxy, Ethoxy, Difluormethoxy, Trifluormethoxy, Fluorethoxy, Chlorethoxy, Methoxyethoxy, Ethoxyethoxy, Methylthio, Ethylthio, Methylsulfinyl, Ethylsulfinyl, Methylsulfonyl, Ethylsulfonyl, Dimethylaminosulfonyl, Phenyl, Methoxycarbonyl, Ethoxycarbonyl, n-Propoxycarbonyl, Isopropoxycarbonyl, n-Butoxycarbonyl, Neopentyloxycarbonyl, Chlorethoxycarbonyl oder Dimethylaminocarbonyl substituiertes Phenyl steht, welches gegebenenfalls einen weiteren Fluor-, Chlor- oder Brom-Substituenten enthält, oder für Pyridinyl steht, welches durch Fluor, Chlor, Brom, Methoxycarbonyl, Ethoxycarbonyl, Dimethylaminocarbonyl, Methylsulfonyl oder Ethylsulfonyl substituiert ist, oder für Thienyl steht, welches durch Chlor, Brom, Methoxycarbonyl oder Ethoxycarbonyl substituiert ist, oder für Pyrazolyl steht, welches durch Chlor, Brom, Methyl, Ethyl, Phenyl, Methoxycarbonyl und/oder Ethoxycarbonyl substituiert ist,

X für Sauerstoff oder Schwefel steht und

Y für Sauerstoff oder Schwefel steht.

**4.** Verfahren zur Herstellung von herbiziden Mitteln, dadurch gekennzeichnet, daß man sulfonylierte (Thio)-Carbamidsäureester gemäß Anspruch 1 mit Streckmitteln und/oder oberflächenaktiven Substanzen vermischt.

**5.** Verfahren zur Bekämpfung von unerwünschten Pflanzen, dadurch gekennzeichnet, daß man sulfonylierte (Thio)Carbamidsäureester gemäß Anspruch 1 auf die unerwünschten Pflanzen und/oder ihren Lebensraum einwirken läßt.

**6.** Verwendung von sulfonylierten (Thio)Carbamidsäureester gemäß Anspruch 1 zur Bekämpfung von unerwünschten Pflanzen.

**7.** Sulfonylierte Carbamidsäureester der allgemeinen Formel (Ia)

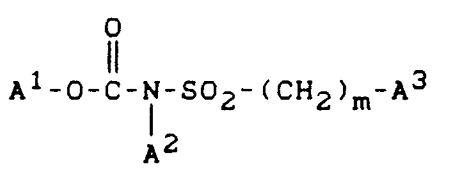

(Ia)

in welcher

m für die Zahlen 0, 1 oder 2 steht,

$A^1$ für jeweils gegebenenfalls substituiertes Alkenyl, Alkinyl, Cycloalkyl, Cycloalkylalkyl, Aryl, Aralkyl, Heterocylcyl oder Heterocyclylalkyl steht,

$A^2$ für Wasserstoff oder für jeweils gegebenenfalls substituiertes Alkyl, Alkenyl, Alkinyl, Cycloalkyl, Cycloalkylalkyl, Aryl, Arylalkyl, Heterocyclyl, Heterocyclylalkyl, für ein Metalläquivalent, für Ammonium, Alkylammonium, Dialkylammonium oder Trialkylammonium steht oder zusammen mit $R^1$ für gegebenenfalls substituiertes Alkandiyl steht, und

$A^3$ für mindestens einmal in ortho-Position substituiertes Phenyl oder für jeweils gegebenenfalls substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht,

mit der Maßgabe, daß die Verbindungen ausgenommen sind, in denen

(1) $A^3$ für 2,4,6-Trimethyl-phenyl oder für gegebenenfalls zusätzlich substituiertes 2-Nitro-phenyl steht und m für die Zahl 0 steht, und

(2) $A^3$ für 2-Chlor-phenyl oder für 2-Methoxycarbonyl-phenyl steht, $A^1$ für Allyl und m für die Zahl 0 steht.

**8.** Sulfonylierte Carbamidsäureester gemäß Anspruch 7, dadurch gekennzeichnet, daß

m für die Zahlen 0, 1 oder 2 steht,

$A^1$ für Alkenyl mit 2 bis 8 Kohlenstoffatomen, Alkinyl mit 2 bis 8 Kohlenstoffatomen, Halogenalkenyl mit 2 bis 8 Kohlenstoffatomen und 1 bis 15 gleichen oder verschiedenen Halogenatomen, oder Halogenalkinyl mit 2 bis 8 Kohlenstoffatomen und 1 bis 13 gleichen oder verschiedenen Halogenatomen steht, oder $A^1$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen, Alkoxycarbonyl mit bis zu 4 Kohlenstoffatomen, oder $A^1$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $A^1$ für jeweils gegebenenfalls einfach bis vierfach, gleich oder verschieden substituiertes Heterocyclyl oder Heterocyclylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, oder Halogenalkyl, Halogenalkoxy oder Halogenalkylthio mit jeweils 1 bis 4 Kohlenstoffatomen und 1 bis 9 gleichen oder verschiedenen Halogenatomen,

$A^2$ für Wasserstoff, Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, Cyanalkyl, Alkoxyalkyl, Alkylthioalkyl, Halogenalkoxyalkyl, Alkoxyalkoxyalkyl, Bis-(Alkoxy)alkyl, Bis-(Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen steht, oder $A^2$ für jeweils gegebenenfalls einfach bis vierfach, gleich oder verschieden substituiertes Cycloalkyl oder Cycloalkylalkyl mit jeweils 3 bis 7 Kohlenstoffatomen im Cycloalkylteil und gegebenenfalls 1 bis 4 Kohlenstoffatomen im Alkylteil steht, wobei als Substituenten im Cycloalkylteil ausgewählt sind: Halogen, Cyano, Alkyl, Alkenyl, Alkinyl oder Alkoxy mit jeweils bis zu 4 Kohlenstoffatomen und Alkoxycarbonyl mit bis zu 4 Kohlenstoffatomen, oder $A^2$ für jeweils gegebenenfalls einfach bis vierfach, gleich oder verschieden substituiertes Phenyl, Naphthyl, Phenylalkyl oder Naphthylalkyl mit gegebenenfalls 1 bis 4 Kohlenstoffatomen in den Alkylteilen steht, wobei als Substituenten im aromatischen Teil ausgewählt sind: Halogen, Cyano, Nitro, Alkyl, Alkoxy, Alkylthio oder

Alkoxycarbonyl mit jeweils 1 bis 4 Kohlenstoffatomen oder Halogenalkyl, Halogenalkoxy oder Halogenalkylthio mit jeweils 1 bis 4 Kohlenstoffatomen und 1 bis 9 gleichen oder verschiedenen Halogenatomen, oder $A^2$ für jeweils gegebenenfalls einfach bis vierfach, gleich oder verschieden substituiertes Heterocyclyl oder Heterocyclylalkyl mit gegebenenfalls 1 bis 4 Kohlenstoffatomen im Alkylteil steht, wobei Heterocyclyl, einzeln oder als Komponente von Heterocyclylalkyl, für Furyl, Thienyl, Oxazolyl, Isoxazolyl, Thiazolyl, Isothiazolyl, Oxadiazolyl, Thiadiazolyl, Pyridinyl, Pyrimidinyl, Tetrahydrofuranyl oder Perhydropyranyl steht und als Substituenten im Heterocyclylteil ausgewählt sind: Halogen, Alkyl, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und 1 bis 9 gleichen oder verschiedenen Halogenatomen, oder

$A^2$ für ein Alkalimetall- oder ein Erdalkalimetall-Äquivalent, für Ammonium, $C_1$-$C_4$-Alkylammonium, Di-($C_1$-$C_4$-Alkyl)-ammonium oder Tri-($C_1$-$C_4$-Alkyl)-ammonium steht oder zusammen mit $A^1$ für gegebenenfalls durch Methyl oder Ethyl substituiertes geradkettiges Alkandiyl mit 2 bis 5 Kohlenstoffatomen steht, und

$A^3$ für mindestens einmal in ortho-Position und insgesamt einfach bis dreifach substituiertes Phenyl oder für jeweils mindestens einfach und gegebenenfalls bis dreifach substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht, wobei jeweils als Substituenten vorzugsweise ausgewählt sind: Halogen, Cyano, Alkyl, Halogenalkyl, Alkoxyalkyl, Alkoxy, Halogenalkoxy, Alkoxyalkoxy, Alkylthio, Halogenalkylthio, Alkylsulfinyl, Halogenalkylsulfinyl, Alkylsulfonyl oder Halogenalkylsulfonyl mit jeweils 1 bis 4 Kohlenstoffatomen in den Alkylteilen und gegebenenfalls 1 bis 9 Halogenatomen, Alkenyl, Halogenalkenyl, Alkinyl oder Halogenalkinyl mit jeweils 2 bis 4 Kohlenstoffatomen und gegebenenfalls 1 bis 7 Halogenatomen, Dialkylaminosulfonyl mit 1 oder 2 Kohlenstoffatomen in den Alkylteilen, Phenyl, Alkoxycarbonyl oder Halogenalkoxycarbonyl mit jeweils 1 bis 5 Kohlenstoffatomen und gegebenenfalls 1 bis 3 Halogenatomen im Alkoxyteil, sowie Dialkylaminocarbonyl mit 1 oder 2 Kohlenstoffatomen in den Alkylteilen.

**9.** Sulfonylierte Carbamidsäureester gemäß Anspruch 7, wobei

m für die Zahlen 0 oder 1 steht,

$A^1$ für Allyl, Propargyl, für jeweils geradkettiges oder verzweigtes Butenyl, Pentenyl, Hexenyl, Butinyl, Pentinyl, Hexinyl oder Heptinyl, für Halogenalkenyl oder Halogenalkinyl mit 3 oder 4 Kohlenstoffatomen und 1 bis 3 Halogenatomen, für jeweils gegebenenfalls einfach bis dreifach, gleich oder verschieden durch Fluor, Chlor, Brom, Methyl, Ethyl, n-Propyl, iso-Propyl, Ethenyl, Propenyl, Ethinyl, Propinyl, Methoxycarbonyl, Ethoxycarbonyl und Cyano substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, für jeweils gegebenenfalls einfach bis dreifach gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl oder Phenylethyl, oder für jeweils gegebenenfalls einfach oder zweifach, gleich oder verschieden durch Fluor, Chlor, Brom, Methyl, Ethyl, Trifluormethyl, Methoxy, Ethoxy und Difluormethoxy substituiertes Furyl, Furylmethyl, Thienyl, Thienylmethyl, Oxazolyl, Oxazolylmethyl, Isoxazolyl, Isoxazolylmethyl, Thiazolyl, Thiazolylmethyl, Isothiazolyl, Isothiazolylmethyl, Oxadiazolyl, Thiadiazolyl, Pyridinyl, Pyrimidinyl, Oxadiazolylmethyl, Thiadiazolylmethyl, Pyridinylmethyl, Pyrimidinylmethyl, Tetrahydrofuranylmethyl oder Perhydrofuranylmethyl steht,

$A^2$ für Wasserstoff, Methyl, Ethyl, n-Propyl, Isopropyl, n-Butyl, Isobutyl, Allyl, Propargyl, für jeweils geradkettiges oder verzweigtes Pentyl, Hexyl, Butenyl, Pentenyl, Hexenyl, Butinyl, Pentinyl, Hexinyl oder Heptinyl, für Halogenalkyl, Halogenalkenyl oder Halogenalkinyl mit bis zu 5 Kohlenstoffatomen und jeweils 1 bis 3 Halogenatomen, für Cyanalkyl, Alkoxyalkyl oder Alkoxycarbonylalkyl mit jeweils 1 bis 4 Kohlenstoffatomen in den einzelnen Alkylteilen, für jeweils gegebenenfalls einfach bis dreifach, gleich oder verschieden durch Fluor, Chlor, Brom, Methyl, Ethyl, n-Propyl, iso-Propyl, Ethenyl, Propenyl, Ethinyl, Propinyl, Methoxycarbonyl, Ethoxycarbonyl und Cyano substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls einfach bis dreifach, gleich oder verschieden durch Fluor, Chlor, Brom, Cyano, Nitro, Methyl, Ethyl, Methoxy, Ethoxy, Methylthio, Ethylthio, Methoxycarbonyl, Ethoxycarbonyl, Trifluormethyl, Trifluormethoxy und Trifluormethylthio substituiertes Phenyl, Benzyl

oder Phenylethyl steht, oder für Natrium, Kalium, Ammonium, Methylammonium, Ethylammonium, n-Proylammonium, Isopropylammonium, Dimethylammonium, Diethylammonium, Dipropylammonium, Diisopropylammonium, Trimethylammonium, Triethylammonium oder Tripropylammonium steht, oder zusammen mit $A^1$ für Dimethylen ($-CH_2CH_2-$) oder Trimethylen ($-CH_2CH_2CH_2-$) steht, und

$A^3$ für in ortho-Position durch Fluor, Chlor, Brom, Cyano, Methyl, Ethyl, Trifluormethyl, Methoxy, Ethoxy, Difluormethoxy, Trifluormethoxy, Fluorethoxy, Chlorethoxy, Methoxyethoxy, Ethoxyethoxy, Methylthio, Ethylthio, Methylsulfinyl, Ethylsulfinyl, Methylsulfonyl, Ethylsulfonyl, Dimethylaminosulfonyl, Phenyl, Methoxycarbonyl, Ethoxycarbonyl, Propoxycarbonyl, Isopropoxycarbonyl, Butoxycarbonyl, Neopentyloxycarbonyl, Chlorethoxycarbonyl oder Dimethylaminocarbonyl substituiertes Phenyl steht, welches gegebenenfalls einen weiteren Fluor-, Chlor- oder Brom-Substituenten enthält, oder für Pyridinyl steht, welches durch Fluor, Chlor, Brom, Methoxycarbonyl, Ethoxycarbonyl, Dimethylaminocarbonyl, Methylsulfonyl oder Ethylsulfonyl substituiert ist, oder für Thienyl steht, welches durch Chlor, Brom, Methoxycarbonyl oder Ethoxycarbonyl substituiert ist, oder für Pyrazolyl steht, welches durch Chlor, Brom, Methyl, Ethyl, Phenyl, Methoxycarbonyl und/oder Ethoxycarbonyl substituiert ist.

**10.** Verfahren zur Herstellung von sulfonylierten Carbamidsäureestern der Formel (Ia)

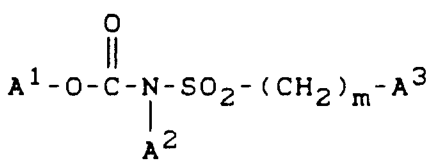

(Ia)

in welcher

m für die Zahlen 0, 1 oder 2 steht,

$A^1$ für jeweils gegebenenfalls substituiertes Alkenyl, Alkinyl, Cycloalkyl, Cycloalkylalkyl, Aryl, Aralkyl, Heterocylcyl oder Heterocyclylalkyl steht,

$A^2$ für Wasserstoff oder für jeweils gegebenenfalls substituiertes Alkyl, Alkenyl, Alkinyl, Cycloalkyl, Cycloalkylalkyl, Aryl, Arylalkyl, Heterocyclyl, Heterocyclylalkyl, für ein Metalläquivalent, für Ammonium, Alkylammonium, Dialkylammonium oder Trialkylammonium steht oder zusammen mit $R^1$ für gegebenenfalls substituiertes Alkandiyl steht, und

$A^3$ für mindestens einmal in ortho-Position substituiertes Phenyl oder für jeweils gegebenenfalls substituiertes Naphthyl, Pyridinyl, Chinolinyl, Furyl, Thienyl, Pyrrolyl, Oxazolyl, Isoxazolyl, Thiazolyl, Isothiazolyl, Imidazolyl oder Pyrazolyl steht,

mit der Maßgabe, daß die Verbindungen ausgenommen sind, in denen

(1) $A^3$ für 2,4,6-Trimethyl-phenyl oder für gegebenenfalls zusätzlich substituiertes 2-Nitro-phenyl steht und m für die Zahl 0 steht, und

(2) $A^3$ für 2-Chlor-phenyl oder für 2-Methoxycarbonyl-phenyl steht, $A^1$ für Allyl und gleichzeitig m für die Zahl 0 steht,

dadurch gekennzeichnet, daß man

(a) Isocyanate der allgemeinen Formel (II)

$O=C=N-SO_2-(CH_2)_m-A^3$ (II)

in welcher

$A^3$ und m die oben angegebene Bedeutung haben,

mit Hydroxyverbindungen der allgemeinen Formel (III) $A^1$-OH (III)

in welcher

$A^1$ die oben angegebene Bedeutung hat,

gegebenenfalls in Gegenwart eines Verdünnungsmittels und gegebenenfalls in Gegenwart eines Reaktionshilfmittels umsetzt, oder daß man

(b) Sulfonsäureamide der allgemeinen Formel (IV)

$H_2N-SO_2-(CH_2)_m-A^3$ (IV)

in welcher
$A^3$ und m die oben angegebene Bedeutung haben,
mit Kohlensäurederivaten der allgemeinen Formel (V)

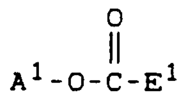

(V)

in welcher
$A^1$ die oben angegebene Bedeutung hat und
$E^1$ für eine elektronenziehende Abgangsgruppe steht,
gegebenenfalls in Gegenwart eines Verdünnungsmittels und gegebenenfalls in Gegenwart eines Reaktionshilfsmittels umsetzt, oder daß man
(c) Sulfonylverbindungen der allgemeinen Formel (VI)

$E^2\text{-}SO_2\text{-}(CH_2)_m\text{-}A^3$ (VI)

in welcher
$A^3$ und m die oben angegebene Bedeutung haben
und
$E^2$ für eine elektronenziehende Abgangsgruppe steht,
mit Carbamidsäureestern der allgemeinen Formel (VII)

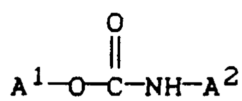

(VII)

in welcher
$A^1$ und $A^2$ die oben angegebene Bedeutung
haben,

gegebenenfalls in Gegenwart eines Verdünnungsmittels und gegebenenfalls in Gegenwart eines Reaktionshilfsmittels umsetzt, oder daß man
(d) sulfonylierte Carbamidsäureester der allgemeinen Formel (Ib)

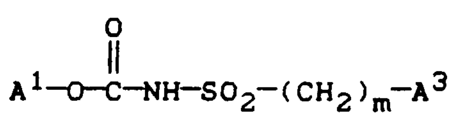

(Ib)

in welcher
$A^1$, $A^3$ und m die oben angegebene Bedeutung
haben,

mit Alkylierungsmitteln der allgemeinen Formel (VIII)

$E^3\text{-}A^2$ (VIII)

in welcher
$A^2$ die oben angegebene Bedeutung hat und
$E^3$ für eine elektronenziehende Gruppe steht,
gegebenenfalls in Gegenwart eines Verdünnungsmittels und gegebenenfalls in Gegenwart eines

Reaktionshilfsmittels umsetzt.

Europäisches Patentamt

# EUROPÄISCHER RECHERCHENBERICHT

Nummer der Anmeldung

**EP 91 11 1297**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-1 340 297 (MAY AND BAKER LTD.)<br>* Seite 7, Spalte rechts, Beispiel 14; Seite 10, Spalte rechts, Beispiele 32,33; Zusammenfassung * | 1-2,4-8, 10 | C 07 C 311/53<br>A 01 N 47/24<br>A 01 N 41/06 |
| X | DD-A-74 982 (W. KOCHMANN et al.)<br>* das ganze Dokument * | 1,2,5-8 | |
| X,D | US-A-3 377 375 (J.A. STEPHENS)<br>* Spalte 2, Zeilen 22-72; Spalte 3 * | 1,2,4-8, 10 | |
| X,D | US-A-3 799 760 (J.A. STEPHENS)<br>* Spalten 2-4,11-13 * | 1,2,4-8, 10 | |
| X | PATENT ABSTRACTS OF JAPAN Band 6, Nr. 239 (C-137)(1117), 26. November 1982;<br>& JP - A - 57140763 (NISSAN) 31.08.1982 (Kat. D) | 1-3,7-10 | |
| X | EP-A-0 185 227 (NIHON TOKUSHU)<br>* Zusammenfassung * | 1-3,7-10 | |
| X | US-A-4 168 152 (E.J. GAUGHAN et al.)<br>* Ansprüche 1,6 * | 1,2 | **RECHERCHIERTE SACHGEBIETE (Int. Cl.5)** |
| X,D | EP-A-0 127 469 (E.I.DU PONT DE NEMOURS & CO.)<br>* Zusammenfassung * | 1-3,10 | C 07 C 311/00<br>A 01 N 47/00<br>A 01 N 41/00 |
| X,D | EP-A-0 101 407 (CIBA GEIGY AG)<br>* Zusammenfassung * | 1-3 | |
| X | PATENT ABSTRACTS OF JAPAN Band 10, Nr. 239 (C-367)(2295), 19. August 1986;<br>& JP - A - 61072745 (NIPPON TOKUSHU) 14.04.1986 | 1-3,5-10 | |

-/-

Der vorliegende Recherchenbericht wurde für alle Patentansprüche erstellt

| Recherchenort | Abschlußdatum der Recherche | Prüfer |
|---|---|---|
| Berlin | 13 August 91 | RUFET J.M.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

**EP 91 11 1297**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-1 668 014 (FARBENFABRIKEN BAYER AG)<br>* Seite 10; Beispiel 6; Ansprüche *<br>– – – | 1-3 | |
| X,D | DE-A-2 644 446 (STAUFFER CHEMICAL CO.)<br>* Ansprüche; Seite 17 *<br>– – – | 1-3,10 | |
| X | FR-A-1 239 917 (FARBWERKE HOECHST AG)<br>* Seite 5; Zusammenfassung *<br>– – – | 1-3 | |
| X | FR-A-2 407 668 (NIPPON SODA)<br>* Anspruch 1 *<br>– – – | 1-3 | |
| X | CHEMICAL ABSTRACTS Band 61, Nr. 9, 26. Oktober 1964, Spalte 10616, Zusammenfassung Nr. 10616g, Columbus, Ohio, US; Z.F. SOLOMKO et al.: "Sulfonanilides. XI. Methyl esters of N-arylsulfonyl-N-phenylcarbamic acid"<br>& Zh. Obshch. Khim., Band 34, Nr. 7, Seiten 2392-2394<br>– – – | 1-3 | |
| X | CHEMICAL ABSTRACTS Band 59, Nr. 3, 5. August 1963, Spalte 2688, Zusammenfassung Nr. 2688f, Columbus, Ohio, US; L.P. GLUSHKO et al.: "Sulfanilides. VII. Ethyl esters of N-arylsulfonyl-N-phenylcarbamic acid"<br>& Zh. Obshch Khim, Band 33, Seiten 612,613<br>– – – | 1-3 | RECHERCHIERTE SACHGEBIETE (Int. Cl.5) |
| X | EP-A-0 125 205 (CIBA-GEIGY AG)<br>* Seiten 25,26; Ansprüche *<br>– – – | 1-3,10 | |
| A | GB-A-2 001 635 (UBE)<br>* Zusammenfassung *; & US-A-4213775 (Kat. D)<br>– – –<br>–/– | 1-10 | |

Der vorliegende Recherchenbericht wurde für alle Patentansprüche erstellt

| Recherchenort | Abschlußdatum der Recherche | Prüfer |
|---|---|---|
| Berlin | 13 August 91 | RUFET J.M.A. |

KATEGORIE DER GENANNTEN DOKUMENTE
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

Europäisches Patentamt

# EUROPÄISCHER RECHERCHENBERICHT

Nummer der Anmeldung

**EP 91 11 1297**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 626 860 (BAYER AG)<br>* Zusammenfassung *<br>– – – | 1-10 | |
| A | AT-A-349 484 (F. HOFFMANN-LA ROCHE)<br>* Seiten 2,3 *<br>– – – – – | 1-3 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| Berlin | 13 August 91 | RUFET J.M.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument